# EUROPEAN PATENT APPLICATION

(11) **EP 4 257 700 A1**
(43) Date of publication of application: **11.10.2023**
(21) Application number: 21899305.3
(22) Date of filing: 02.12.2021
(51) Int. Cl.: C12Q 1/42, C12M 1/34, G01N 33/50

(54) **SELECTIVE FUNCTIONAL SUBSTANCE SEARCHING METHOD, SELECTIVE FUNCTIONAL SUBSTANCE SEARCHING DEVICE, SELECTIVE FUNCTIONAL SUBSTANCE SEARCHING METHOD, AND PROGRAM**

(30) Priority: 04.12.2020 JP 2020201892
(71) Applicant: Shinshu University, Matsumoto-shi, Nagano 390-8621 (JP)
(72) Inventor: KII, Isao, Kamiina-gun, Nagano 399-4598 (JP); FURUIE, Gaku, Kamiina-gun, Nagano 399-4598 (JP); UMEZAWA, Koji, Kamiina-gun, Nagano 399-4598 (JP)
(74) Representative: Grünecker Patent- und Rechtsanwälte PartG mbB
(86) International application number: PCT/JP2021/044375
(87) International publication number: WO 2022/118941

(57) **Abstract**

Destabilize a protein at least partially, provide the destabilized protein in a presence of a candidate functional substance, induce its restabilization, and determine the effect of a presence of the candidate functional substance in influencing a function of the protein, in order to search for functional substances that selectively bind to a non-native state of the protein and influence the function of the protein.

## Description

### Technical Field

The present invention relates to a method of searching for selective functional substances, a selective functional substance search system, a selective functional substance search method, and a program.

### Background Art

Drugs and other useful compounds are discovered by evaluating and searching millions of compounds using, as an indicator, how these compounds could inhibit the functions of proteins associated with development of diseases. Many cancers, mental disorders, adult-onset diseases, etc., enhance the activity of an enzyme, etc., causing the disease increases due to genetic mutation or effects of environmental factors. Accordingly, drugs exhibit their efficacy when the compound being the primary component thereof binds to the target protein associated with the disease and suppresses its activity. Such compounds are essential to medicine, and the first step of any drug development is to evaluate and search for compounds that selectively bind to a specific protein.

For example, Non-patent Literature 1 discloses that candidate inhibitor compounds with inhibitory activity are being evaluated targeting 321 types of kinase proteins.

Also, Non-patent Literature 2 discloses a screening system, representing a compound screening using cultured cells for detecting a compound's pharmacological action as an intracellular signal change.

Also, Non-patent Literature 3 discloses evaluating compounds using a cell-free protein translation system.

### Background Art Literature

### Non-patent Literature

Non-patent Literature 1: Carna Biosciences Inc., "Profiling Service - Evaluation of Inhibitory Activity against 321 Types of Complete Kinases," [online], publication date unknown, [searched November 20, 2020], Internet <URL: https://www.carnabio.com/japanese/product/search.cgi?mode=profiling>
Non-patent Literature 2: Carna Biosciences Inc., "NanoBRET TE Intracellular Kinase Cell-based Assay Service," [online], publication date unknown, [Searched November 20, 2020], Internet <URL: https://www.carnabio.com/japanese/product/nanobret-sevices.html>
Non-patent Literature 3: GeneFrontier Corporation, "PUREfrex Reconstituted Cell-free Protein Synthesis Kit," [online], publication date unknown, [Searched November 20, 2020], Internet <URL: https://www.genefrontier.com/solutions/purefrex/>

### Summary of the Invention

### Problems to Be Solved by the Invention

However, while approximately 20,000 types of proteins are synthesized in human body cells, only thousands of them can become the targets of compounds, and also because the compound binding sites of these proteins are limited to just one or two locations per protein, it is nearly impossible to address the astronomical number of compounds in terms of structural diversity; as a result, the conventional search methods present a problem in that the probability of discovering a useful functional substance is very low.

The present invention was made in light of the aforementioned problem, and an object of the present invention is to provide a method of searching for selective functional substances, a selective functional substance search system, a selective functional substance search method, and a program, that can bridge the huge gap between the diversity of compounds and the number of protein binding sites to increase the probability of discovering a useful functional substance.

### Means for Solving the Problems

To achieve the aforementioned object, the method of searching for selective functional substances proposed by the present invention is a method of searching for selective functional substances to search for functional substances that selectively bind to a non-native state of a protein and influence the function of the protein, including: a step to destabilize the protein at least partially; a step to induce restabilization by providing the destabilized protein in the presence of a candidate functional substance to; and a step to determine the effect of the presence of the candidate functional substance in influencing the function of the protein.

Also, the selective functional substance search system proposed by the present invention is a selective functional substance search system comprising at least a memory part and a control part, to search for functional substances that selectively bind to a non-native state of a protein and influence the function of the protein, wherein: the memory part stores structure data relating to at least one candidate functional substance; and the control part comprises: a destabilization part that destabilizes the protein at least partially, through simulation and provides the destabilized protein in the presence of the candidate functional substance; and a determination part that determines the effect of the presence of the candidate functional substance in influencing the function of the protein.

Also, the selective functional substance search method proposed by the present invention is a selective functional substance search method to be executed by a computer comprising at least a memory part and a control part, in order to search for functional substances that selectively bind to a non-native state of a protein and influence the function of the protein, wherein: the memory part stores structure data relating to at least one candidate functional substance; and the method includes the following executed in the control part: a destabilization step to destabilize the protein at least partially and provide the destabilized protein in the presence of the candidate functional substance, through simulation; and a determination step to determine the effect of the presence of the candidate functional substance in influencing the function of the protein.

Also, the program proposed by the present invention is a program to be executed by a computer comprising at least a memory part and a control part, in order to search for functional substances that selectively bind to a non-native state of a protein and influence the function of the protein, wherein: the memory part stores structure data relating to at least one candidate functional substance; and the program is designed to execute in the control part: a destabilization step to destabilize the protein at least partially, through simulation, and provide the destabilized protein in the presence of the candidate functional substance; and a determination step to determine the effect of the presence of the candidate functional substance in influencing the function of the protein.

Additionally, in the aforementioned method of searching for selective functional substances, selective functional substance search system, selective functional substance search method, or program under the present invention, a solution containing the protein is subjected to a temperature change, a pressure change, a pH change or an addition of denaturant, or an alteration of electric-charge on the protein, for the destabilization.

Additionally, in the aforementioned method of searching for selective functional substances, selective functional substance search system, selective functional substance search method, or program under the present invention, the protein is a kinase, protease, or other enzyme, photoprotein, or other functional protein.

Additionally, in the aforementioned method of searching for selective functional substances, selective functional substance search system, selective functional substance search method, or program under the present invention, "destabilize the protein" means subjecting it to temperature change, where the destabilizing temperature is 50°C to 70°C.

Additionally, in the aforementioned method of searching for selective functional substances, selective functional substance search system, selective functional substance search method, or program under the present invention, the protein is destabilized by means of the temperature change for a specified heating temperature period and/or cooled to induce restabilization of the protein.

Additionally, in the aforementioned method of searching for selective functional substances, selective functional substance search system, selective functional substance search method, or program under the present invention, the function of the protein is exhibited in the presence of a substrate or binding substance.

Additionally, in the aforementioned method of searching for selective functional substances, selective functional substance search system, selective functional substance search method, or program under the present invention, activity of the candidate functional substance with respect to the destabilized protein is detected in a dynamic range of 1.5 times or more in the presence of a surfactant or hydrotrope.

Additionally, in the aforementioned method of searching for selective functional substances, selective functional substance search system, selective functional substance search method, or program under the present invention, the candidate functional substance is a small molecule, middle molecule, large molecule, peptide, antibody, or nucleic-acid aptamer.

Additionally, in the aforementioned method of searching for selective functional substances, selective functional substance search system, selective functional substance search method, or program under the present invention, the functional substance is an inhibitor, accelerator, coagulant, stabilizer, or activator of the protein. Here, an "inhibitor" may mean a substance that, for example, binds to a non-native state of the target protein and inhibits the function of the protein. Also, an "accelerator" may mean a substance that, for example, binds to a non-native state of the target protein and accelerates the function of the protein. Also, a "coagulant" may mean a substance that, for example, binds to a non-native state of the target protein and induces coagulation of the protein. Also, a "stabilizer" may mean a substance that, for example, binds to a non-native state of the target protein and stabilizes the protein, thereby improving the function of the protein. Also, an "activator" may mean a substance that, for example, binds to a non-native state of the target protein and activates the function of the protein.

### Effects of the Invention

According to the present invention, a method of searching for selective functional substances, a selective functional substance search system, a selective functional substance search method, and a program, can be provided, that can bridge the huge gap between the diversity of compounds and the number of protein binding sites to increase the probability of discovering a useful functional substance.

### Brief Description of the Drawings

[Fig. 1] FIG. 1 is a figure showing a conceptual overview of the embodiment pertaining to the present invention.
[FIG. 2] FIG. 2 is a figure showing the mechanism of how peptides are folded into a complete protein, as well as an example of inhibitor with folding intermediates.
[FIG. 3] FIG. 3 is a figure showing an example of reproducing a folding intermediate structure.
[FIG. 4] FIG. 4 is a graph showing the inhibitory effect of the inhibitor FINDY on the reproduced folding intermediate structure.
[FIG. 5] FIG. 5 is a block diagram showing an example of the selective functional substance search system 100 to which the embodiment is applied.
[FIG. 6] FIG. 6 is a flowchart showing an example of the selective functional substance search method using the destabilization part 102a and determination part 102b of the selective functional substance search system 100.
[FIG. 7A] FIG. 7A is a figure showing the inhibitory effects of heating/quick cooling on complete enzymatic activity in two types of specific inhibitors with the folding intermediate.
[FIG. 7B] FIG. 7B is a figure showing that the inhibitory effects of heating/quick cooling on complete enzymatic activity in two types of specific inhibitors with the folding intermediate are concentration-dependent.
[FIG. 7C] FIG. 7C is a figure showing the inhibitory effects measured using the known substance RD0392 and the known substance Harmine as controls.
[FIG. 7D] FIG. 7D is a figure showing the test results in cultured cells.
[FIG. 7E] FIG. 7E is a figure showing the inhibitory effect of heating/quick cooling on complete enzymatic activity by specific inhibitor compound No. 2 with the folding intermediate.
[FIG. 7F] FIG. 7F is a figure showing the western blotting detection results with compound No. 2.
[FIG. 7G] FIG. 7G is a figure showing the small-scale inhibitor/activator screening results with respect to the kinase DYRK1A.
[FIG. 7H] FIG. 7H is a figure showing the inhibitory effect of compound No. 20 identified as an inhibitor in the embodiment as a result of screening.
[FIG. 8A] FIG. 8A is a graph showing the relationship of ATP concentration and inhibitory effect.
[FIG. 8B] FIG. 8B is graphs showing the relationships between concentrations of ADP, GDP, sodium xylene sulfonate and sodium p-toluenesulfonate, and inhibitory effects.
[FIG. 9] FIG. 9 is a figure showing the inhibitory activities without heating/cooling attributable to three types of specific inhibitors FINDY, 168, and No. 2 with the folding intermediate.
[FIG. 10A] FIG. 10A is a figure showing the inhibitory activities with heating/cooling attributable to three types of specific inhibitors FINDY and 168 with the folding intermediate.
[FIG. 10B] FIG. 10B is a figure showing that the inhibitory effects of heating/quick cooling on complete enzymatic activity in two types of specific inhibitors with the folding intermediate are concentration-dependent.
[FIG. 10C] FIG. 10C is a figure showing the inhibitory effects measured using the known substance RD0392 and the known substance Harmine as controls.
[FIG. 10D] FIG. 10D is a figure showing the inhibitory activities of three types of specific inhibitors FINDY, 168, and No. 2 with the folding intermediate, with and without heating/cooling, on the kinase SRC.
[FIG. 10E] FIG. 10E is a figure showing the inhibitory activities of three types of specific inhibitors FINDY, 168 and No. 2 with the folding intermediate, with and without heating/cooling, on the kinase ABL.
[FIG. 11] FIG. 11 is a figure showing the temperature *t*1 at which the enzymatic activity of the protein was lost in a constantly heated state.
[FIG. 12] FIG. 12 is a figure showing the temperature t2 at which the enzymatic activity was lost even when quick cooling was performed after heating for a relatively short period of 20 seconds.
[FIG. 13] FIG. 13 is a figure showing the temperature t between the temperature *t*1 at which deactivation occurs after a long period and the temperature t2 at which deactivation occurs even after a short period of heating followed by quick cooling (*t*1<*t*<*t*2).
[FIG. 14] FIG. 14 is a graph showing the inhibitory effects under various temperature conditions.
[FIG. 15A] FIG. 15A is a graph showing the inhibitory effects under various temperature conditions.
[FIG. 15B] FIG. 15B presents data expressing FIG. 14 and FIG. 15A in an easier-to-understand manner.
[FIG. 15C] FIG. 15C is a figure showing the relative enzymatic activities of kinase DYRK1A, under heating at a constant temperature (no cooling step).
[FIG. 15D] FIG. 15D is a figure showing the inhibitory effects, at varying rates of cooling, with respect to the kinase DYRK1A.
[FIG. 16] FIG. 16 is a figure showing the study results on upper-limit temperature and lower-limit temperature for the kinase DYRK1A.
[FIG. 17] FIG. 17 is a figure showing the study results on upper-limit temperature and lower-limit temperature for the kinase DYRK1B.
[FIG. 18] FIG. 18 is a figure showing the study results on upper-limit temperature and lower-limit temperature for the kinase SRC.
[FIG. 19] FIG. 19 is a figure showing the study results on upper-limit temperature and lower-limit temperature for the kinase ABL.
[FIG. 20] FIG. 20 is a figure showing the study results on upper-limit temperature and lower-limit temperature for the monoamine oxidase MAO-A.
[FIG. 21] FIG. 21 is a figure showing the small-scale inhibitor screening results with respect to the monoamine oxidase MAO-A.
[FIG. 22] FIG. 22 is a figure showing the study results on upper-limit temperature and lower-limit temperature for the protease Calpain-I.
[FIG. 23] FIG. 23 is a figure showing the small-scale inhibitor screening results with respect to the protease Calpain-I.
[FIG. 24] FIG. 24 is a figure showing the screening results pertaining to a separate small-scale structural analogue library (Nos. 1 to 26).
[FIG. 25] FIG. 25 is a figure showing (1), "Calculation system and initial structure of DYRK1A-FINDY."
[FIG. 26] FIG. 26 is a schematic drawing of the temperature settings in (2), "Setup of MD simulation with temperature jump."
[FIG. 27] FIG. 27 is a figure showing the simulation results with each heating temperature set to 400K.
[FIG. 28] FIG. 28 is a figure showing the simulation results with each heating temperature set to 450K.
[FIG. 29] FIG. 29 is a figure showing the simulation results with each heating temperature set to 500K.
[FIG. 30] FIG. 30 is a figure showing the simulation results with each heating temperature set to 550K.
[FIG. 31] FIG. 31 is a figure showing the simulation results with each heating temperature set to 600K.
[FIG. 32] FIG. 32 is a figure showing the simulation results with each heating temperature set to 600K for only ligands.
[FIG. 33] FIG. 33 is a figure showing the simulation results with each heating temperature set to 800K for only ligands.
[FIG. 34] FIG. 34 is a figure showing the simulation results with each heating temperature set to 1000K for only ligands.
[FIG. 35] FIG. 35 is quantitative graphs of jumping the temperature for all in the system.
[FIG. 36] FIG. 36 is quantitative graphs of jumping the temperature for only ligands.

### Mode for Carrying Out the Invention

A mode for carrying out the method of searching for specific functional substances, selective functional substance search system, selective functional substance search method, and program, as well as recording media, pertaining to the embodiment provided herein of the present invention, is explained below in detail based on the drawings. It should be noted that the present invention is not limited by this embodiment.

An overview, constitution and processing, as well as examples, etc., of the embodiment pertaining to the present invention are explained below in detail by referring to various examples. It should be noted that, in the explanations below, a description of numerical range such as "50°C to 70°C" is interpreted to include the lower limit and the upper limit. Also, a technique described as being performed manually may be performed automatically, or conversely a technique described as being performed automatically may be performed manually, or these modes may be combined as desired. Similarly, a technique described as being implemented in vivo, in vitro, etc., may be implemented in silico, etc., by means of simulation on a computer, or conversely a technique described as being implemented in silico, etc., by means of simulation may be implemented in vivo, in vitro, etc., or these modes may be combined as desired.

### [Overview of the Embodiment]

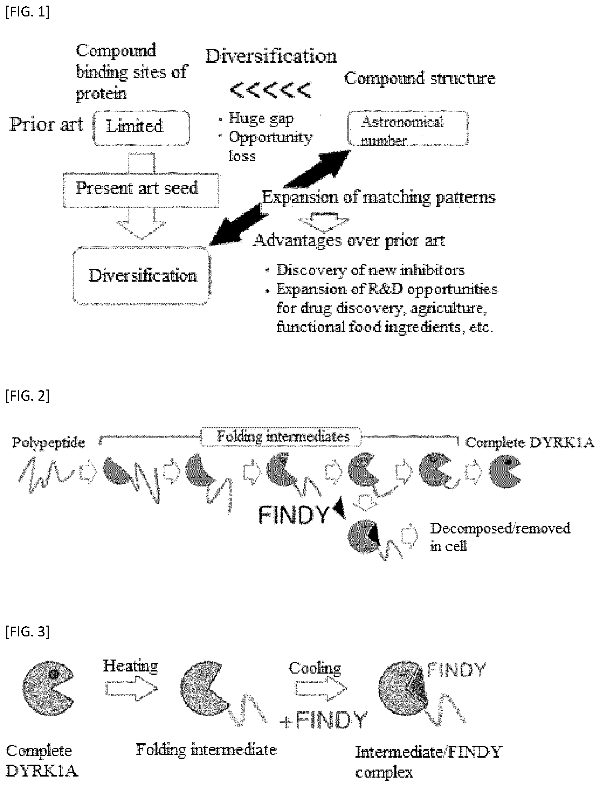

First, an overview of the embodiment pertaining to the present invention is explained. FIG. 1 is a drawing showing a conceptual overview of the embodiment pertaining to the present invention.

As described above, while approximately 20,000 types of proteins are synthesized in human body cells, only thousands of them can become the targets of compounds, and also the compound binding sites of these proteins are extremely limited. Meanwhile, there are, in terms of structural diversity, an astronomical number of compounds that can become candidate functional substances, and because of this huge gap between the two, the conventional methods present a problem in that the probability of discovering a useful functional substance is extremely low. A more specific example of the challenge is seen in the problem below.

Essentially, with many of the compound binding sites (binding pockets) of the proteins identified so far, the structure is preserved according to the type of protein. With a kinase, for example, the site (pocket) bound by adenosine triphosphate (ATP) represents one such site. To the extent that their targets are these binding pockets whose structure is preserved, obtaining selective inhibitors and other functional substances has been difficult. Also, inhibitors and other functional substances targeting these binding pockets whose structure is preserved exhibit activity against proteins other than those associated with diseases, thereby presenting a problem of side effects. (Reference: https://www.ncbi.nlm.nih.gov/pmc/articles/PMC7527212/, https://www/ncbi.nlm.nih.gov/pmc/articles/PMC6088748/, https://www.sciencedirect.com/science/article/abs/pii/S0969212619303910?via%3Dihu b;
reference on hidden binding sites (sites bound by compounds appearing in non-native state, not native state): https://www.amed.go.jp/news/seika/kenkyu/20201005.html).

The inventors of the invention under the present application for patent studied in earnest in light of the aforementioned problem, and ultimately devised the present invention including the embodiment provided herein.

To be specific, the embodiment, which represents an embodiment of the present invention, is a method of searching for selective functional substances to search for functional substances (inhibitors, etc.) that selectively bind to a non-native state of a protein and influence the function of the protein, wherein, firstly, the protein is destabilized at least partially (step A). As a result, the protein changes from a native state in stable state to a non-native state in metastable state.

Here, a "native state" may be defined as, for example, the most stable structure associated with the lowest amount of free energy. Meanwhile, as an example, a non-native state can appear in a cell during, for example, a process where peptides are folded into a higher-order structure, or a process where structural change in protein is accelerated by ubiquitin, etc., due to exposure to high heat in the case of burn injury, etc. It should be noted that the folding process may be defined as a process where peptides are folded sterically into a protein of higher-order, three-dimensional structure, for example. It should be noted that a refolding process that achieves restabilization may be defined, for example, as a process where the three-dimensional structure of the protein fluctuates in an attempt to revert to its original state. It should be noted that the embodiment may be implemented by reading "selective" as "specific," or vice versa. It should be noted that, as an example, "selective" to *a* may be defined as referring to a state where *a* is more likely selected than *b,* for example, while "specific" to *a,* to a state where only *a* is the target and *b,* etc., other than *a* will not be the target.

Also, as an example, destabilization involves any one of processes where the solution containing the protein is subjected to temperature change (e.g.: heating at 40°C to 100°C, or preferably 50°C to 70°C), pressure change (e.g.: 200 to 400 MPa), pH change (e.g.: pH2 to pH12), addition of denaturant (e.g.: urea, guanidine hydrochloride, sodium dodecyl sulfate or other surfactant, dimethyl sulfoxide or other organic solvent), or alteration of electric-charge on the protein (e.g.: reducing the protein charge, which is 100% under physiological conditions, to an arbitrary value as low as 1%), where these processes may be used in combination. There may be a process where the protein is reverted to its initial state to induce restabilization. If temperature change by means of heating is adopted as a form of destabilization, for example, the temperature may be cooled to achieve restabilization. In the case of pressure change, the pressurized state should be brought back to the depressurized state. Although the rate at which the restabilization process is implemented is not limited, the process time should be shortened in order to increase the throughput.

When destabilizing through temperature change by means of heating, heating should be performed at the temperature, and for the period, needed to turn the protein into an unstable state, where preferably adjustments such as raising the temperature while shortening the period are made. Heating should be performed for at least 1 second, or preferably 3 seconds or more, if the temperature is 50°C to 70°C, and the heating conditions should be selected to the extent that the enzymatic activity will not be lost as a result of heating too long or at too high temperature.

Also, the target protein may be any protein such as kinase, protease and other enzyme, photoprotein, or other functional protein. Kinases include, for example, the CMGC family under which DYRK1A/GSK3 associated with neurological diseases are classified, the TK family under which ABL/EGFR associated with cancers and JAK associated with immunological diseases are classified, the TKL family under which BRAF/AKT associated with cancers are classified, the STE family under which MAPK associated with cancers is classified, the CK1 family under which CK associated with in vivo regulation is classified, the AGC family under which S6K/PKC associated with cancers are classified, and the CAMK family under which CHK associated with cancers and CaMK associated with neurological diseases are classified. To be more specific, kinases may be the kinase DYRK1B, kinase SRC, and kinase ABL. Proteases and other enzymes include, for example, the cancer-associated matrix metalloproteinases MMP, cancer-associated protease BMP-1, angiotensin-converting enzymes, virus-associated protease HIV-PR, caspases, cathepsins, protease Calpain-I, neurological disease-associated secretases, bacteria-carrying proteases, and parasite-carrying proteases. Photoproteins include, for example, firefly-derived luciferase, shrimp-derived luciferase, and fungus-derived luciferase. Other functional proteins include, for example, virus-derived polymerase, G-protein coupled receptors, monoamine oxidase MAO-A, ion channels, tubulins/actins and other structural proteins, RNA/DNA polymerases, phosphatases, ubiquitin ligases, deubiquitinating enzymes, proteasomes, NPC (nuclear pore complex) component proteins, membrane receptor proteins, cytokines, molecular chaperones, transporters, integrins, nuclear receptors, esterases, metabolizing enzymes, and viral envelope proteins.

Next, in the embodiment, the destabilized protein is provided in the presence of a candidate functional substance (step B). Here, the candidate functional substance may coexist with the protein before destabilization, or it may coexist with the protein during or after destabilization. The candidate functional substance represents any substance such as small molecule, middle molecule, large molecule, peptide, antibody, or nucleic-acid aptamer. The candidate functional substance may be used in an amount of at least 1 mol per 1 mol of the protein, and in terms of concentration in the reaction solution, the candidate functional substance may be used at a concentration of 1 picomol/L to 1 mol/L. It should be noted that, furthermore in the embodiment, restabilization of the destabilized protein may be induced. Also, a substrate, binding substance, surfactant, hydrope, etc., may coexist with the protein before or after protein destabilization. A substrate is known as a substance that triggers chemical reaction when subjected to the action of an enzyme, and specific examples include peptides and proteins that bind to a kinase to cause the phosphorylated groups to transfer from ATP due to the kinase. Peptides and proteins that bind to a protease to sever the internal sequence are also included. Compounds that bind to a modifying enzyme to change its structure are also included. A surfactant may be defined as a substance having a lipophilic group and a hydrophobic group, while a hydrope may be defined as a substance having a hydrophilic group and a hydrophobic group in a molecule and characterized in that it causes a protein or other organic compound to dissolve, at high concentration, in water or in an aqueous solution of salt. Examples of hydrotropes include adenosine triphosphate (ATP), adenosine diphosphate (ADP), sodium p-toluenesulfonate, guanosine triphosphate (GTP), urea, tosylate, sodium cumene sulfonate, and sodium xylene sulfonate. Hydrotropes and surfactants may be used in the reaction solution at a concentration of 1 nanomol/L to 1 mol/L. Any material required in these reactions may be used by dissolving it with a necessary solvent. Solvents that can be used include DMSO, THF, DMF, etc.

Lastly, in the embodiment, the effect of the presence of the candidate functional substance in influencing the function of the protein is determined (step C). For example, what is determined is, such as, inhibitory effect when searching for an inhibitor, accelerative effect when searching for an accelerator, coagulative effect when searching for a coagulant, stabilizing effect when searching for a stabilizer, or activating effect when searching for an activator, as a functional substance. If the function of the protein is influenced (by a function inhibitory effect, for example) by 1.5 times or more (or preferably twice or more; it should be noted that an inhibitory effect of 10 times or more was achieved in the Examples) due to the presence of the candidate functional substance, then the substance can be detected as a useful functional substance. As an example, in this process a determination can be made by using the Promega Kinase Glo assay kit, etc., to quantify the amount of ATP remaining in the tube, and thereby measuring its enzymatic activity. It should be noted that the determination of inhibitory effect is not limited to one based on use of the aforementioned kit, etc.

The foregoing provided an overview of the embodiment pertaining to the present invention. The embodiment may be implemented manually including in vivo, in vitro, etc., or automatically (via automation), or by means of simulation.

As described above, destabilizing a protein diversifies the structure and binding site of the protein to increase the probability of matching the protein to a group of substances that can serve as functional substances, thereby increasing the possibility of discovering a new drug, etc. (refer to FIG. 1).

Now, the principle behind why the aforementioned effects are achieved by the embodiment of the present invention is explained. FIG. 2 is a drawing showing the mechanism of how peptides are folded into a complete protein, as well as an example of folding intermediate inhibitor.

The inventors of the invention under the present application for patent had discovered, prior to the present invention, an art that would constitute a preliminary stage toward diversification, as shown in FIG. 2. Under this art, it was demonstrated that a transient transition state (folding intermediate) appearing during the course of peptides folding into and maturing as a protein (kinase DYRK1A in this example) could become the target of a compound (inhibitor FINDY in this example) (Kii et al. Nat Commun 2016; 10).

By studying in earnest based on this finding, the inventors of the invention under the present application for patent discovered the principle behind the embodiment of the present invention, which is that the diverse structures presented by folding intermediates could dramatically increase the compound binding sites of proteins, thereby dramatically increasing the chances of drug discovery.

And, as a result of further study, the inventors of the invention under the present application for patent ultimately established an art of reproducing, in vitro in a simplified manner, a folding intermediate structure that will exist only temporarily and for a very short period of time in a cell. FIG. 3 is a drawing showing an example of reproducing a folding intermediate structure.

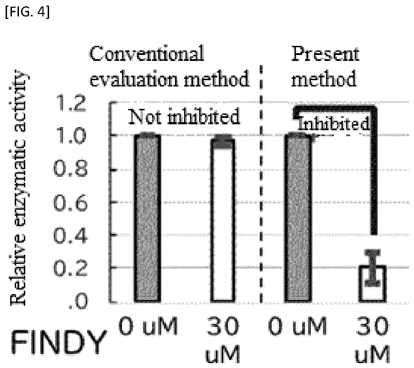

Specifically, the inventors of the invention under the present application for patent discovered that by heating a complete protein, it could be transitioned from a stable state to a metastable state so that the folding process of the protein could be reproduced, as shown by way of example in FIG. 3. Now, FIG. 4 is a graph showing the inhibitory effect of the inhibitor FINDY on the reproduced folding intermediate structure. It should be noted that, for the test conditions, etc., for confirming the inhibitory effect, the aforementioned paper (Kii et al. Nat Commun 2016; 10) is to be referenced.

As shown in FIG. 4, it was demonstrated that, to the folding intermediate structure reproduced by the heating/cooling process (this technique) representing an example of the protein destabilization in the embodiment, FINDY, which is a specific inhibitor with the folding intermediate, had actually bound, and that functional inhibition occurred as a result. It should be noted that, in a functional inhibition test of the complete protein (conventional evaluation method), functional inhibition was confirmed to be non-existent.

As described above, in the embodiment a protein is destabilized by a process of heating/cooling, etc., and thus transitioned from a stable state to a metastable state, thereby artificially creating diverse structures of the protein to be targeted and allowing the target protein to present diverse binding sites for a compound, which expands the compound matching patterns and increases the efficiency of drug discovery, etc. It should be noted that the aforementioned destabilization method by a heating/cooling process is one example and the embodiment also encompasses destabilizing a protein by various methods such as a temperature change, a pressure change, a pH change, an addition of denaturant, and/or an alteration of electric-charge on protein.

Also, while the above explanation uses the kinase DYRK1A as an example of the target protein, the embodiment can be applied to all proteins in light of its principle. In other words, it can be applied to enzymes other than kinases as well as various proteins other than enzymes. This means that, according to the embodiment, compound binding sites may appear in the folding process even on proteins that otherwise have no compound binding sites and were therefore excluded from the target of drug discovery, and this also contributes to discovery of new targets.

Also, while discovery of drugs, etc., is the purpose in the above explanation, the functional substances to be obtained by the embodiment can also be utilized, in addition to drug discovery, in the creation of agrochemicals specific to the proteins to be removed in plants, pests, pathogenic bacteria, etc., or of functional ingredients for food, etc., having a new mechanism of action not dependent on antioxidation. It should be noted that, while the embodiment is explained primarily by using an inhibitor as an example of the functional substance to search for, this has no limiting effect and the present invention can also be applied in the search for such functional substances as accelerators, coagulants, stabilizers, and activators.

### [Constitution of Selective Functional Substance Search System]

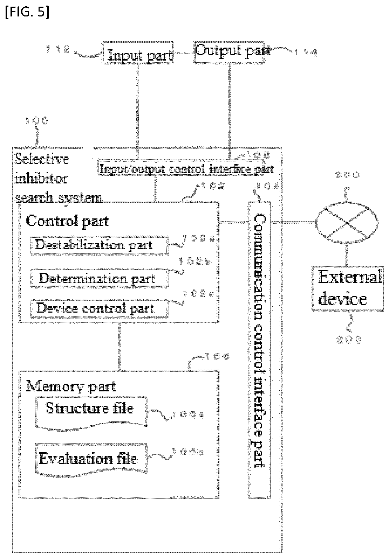

Next, the constitution of a selective functional substance search system 100 representing an example of a mode for carrying out the aforementioned embodiment by means of simulation, is explained by referring to FIG. 5. It should be noted that the items described below can be applied to either manual or automatic methods. FIG. 5 is a block diagram showing an example of the selective functional substance search system 100 to which the embodiment is applied, mainly showing, in concept, those parts of the constitution that relate to the embodiment.

As shown in FIG. 5, the selective functional substance search system 100 in the embodiment comprises, roughly, at least a control part 102 and a memory part 106, and in the embodiment it further comprises an input/output control interface part 108 and a communication control interface part 104.

Here, the control part 102 is a CPU, etc., that controls the selective functional substance search system 100 as a whole in an integrated manner. Also, the communication control interface part 104 is an interface connected to a router or other communication unit (not illustrated) to be connected to a communication line, etc., while the input/output control interface part 108 is an interface connected to an input part 114 and an output part 116. Meanwhile, the memory part 106 is a unit for storing various types of databases, tables, etc. Each of these parts of the selective functional substance search system 100 is connected in a communication-ready manner via an arbitrary communication path. Furthermore, this selective functional substance search system 100 is connected to a network 300 in a communication-ready manner via a router or other communication unit as well as a dedicated line or other wired or wireless communication line.

The various databases and tables (in a structure file 106a, etc.) stored in the memory part 106 represent a storage means for fixed disk units, etc. For example, the memory part 106 stores various programs, tables, files, databases, webpages, etc., to be used in various processes.

Of these various constituents of the memory part 106, the structure file 106a provides a structure data memory means for storing structure data. Structure data may be structure data of target proteins, structure data of candidate functional substances and other substances, or structure data of water molecules and solutes, protein substrates and binding substances, surfactants, hydropes, and so on. In the structure file 106a, structure data, etc., obtained via the input part 114, network 300, etc., may also be stored. It should be noted that the structure data in the structure file 106a includes, for example, the coordinates of each atom in the two-dimensional space or three-dimensional space.

In FIG. 5, the input/output control interface part 108 controls the input part 114 and output part 116. Here, a monitor, speaker or printer may be used as the output part 116. Meanwhile, a keyboard, mouse, microphone, etc., may be used as the input part 114. It should be noted that these are not the only examples and the output part 116 may be a destabilization means/restabilization means capable of performing heating/cooling, etc., such as a PCR unit, etc., while the input part 114 may be a determination means capable of determining the functionality of proteins using fluorescent pigments, etc., such as a real-time PCR unit, etc.

Also, in FIG. 5, the control part 102 has an internal memory for storing an OS (operating system) or other control program, programs specifying various processing procedures, etc., and required data. And, the control part 102 processes information to execute various processes using these programs, etc. The control part 102, in functional concept, comprises a destabilization part 102a, a determination part 102b, and a device control part 102c.

Of these, the destabilization part 102a is a destabilization means for destabilizing a protein at least partially based on the structure data stored in the structure file 106a, and providing the destabilized protein in the presence of a candidate functional substance, by means of simulation.

As an example, the destabilization part 102a may achieve destabilization by causing, by means of simulation, a temperature change, a pressure change, pH change, an addition of denaturant, and/or an alteration of electric-charge on protein in a solution based on the three-dimensional structure data of the target protein stored in the structure file 106a. For example, the destabilization part 102a may perform a simulation where the speed of the atoms constituting the structure is changed to reflect a temperature change/pressure change. Also, the destabilization part 102a may perform a simulation where the potential energy is changed to reflect an electrostatic interaction/van der Waals' interaction/binding energy change. Additionally, the destabilization part 102a not only destabilizes the target protein directly, but it may also destabilize the target protein indirectly by destabilizing the surface of the target protein. For example, destabilization may be achieved by subjecting the water molecules, substrate, binding substance, candidate functional substance, surfactant, hydrope, etc., around the target protein to temperature change, etc., thereby heating or otherwise manipulating the target protein indirectly from the surface.

Also, the destabilization part 102a, as an example, causes the three-dimensional structure of the destabilized protein to interact, in the simulation, with the three-dimensional structure based on the candidate functional substance data stored in the structure file 106a. It should be noted that, in the simulation, the candidate functional substance may be added before or after the protein is destabilized, and a restabilization process that is a reverse process of destabilization may also be added. Additionally, destabilization may involve performing a simulation that has been modified partially or entirely not only for the protein, but also for the candidate functional substance, solvent molecule, etc., in coexistence therewith. It should be noted that, for the three-dimensional structure destabilization method or interactive simulation performed by the destabilization part 102a, any known technique such as in silico screening, etc., may be utilized.

Also, as shown in FIG. 5, the determination part 102b serves, in the simulation performed by the destabilization part 102a, as a determination means for determining the effect of the presence of the candidate functional substance in influencing the function of the protein. For example, the determination part 102b may determine inhibitory effect when searching for an inhibitor, accelerative effect when searching for an accelerator, coagulative effect when searching for a coagulant, stabilizing effect when searching for a stabilizer, or activating effect when searching for an activator, as a functional substance. As an example, the determination part 102b may determine functionality influencing effect (such as inhibitory effect, accelerative effect, stabilizing effect, activating effect, etc., on the function of the protein) according to the degree of fitting of the candidate functional substance with the three-dimensional structures (native state and non-native (destabilized) states) of the protein. For example, if the candidate functional substance is a weak fit with the native state of the protein but a strong fit with a non-native state (destabilized structure) of the protein that has been destabilized by the destabilization part 102a, then the determination part 102b may determine that the candidate functional substance has an effect of selectively binding to the non-native state of the protein and influencing its function (i.e., function influencing effect). It should be noted that the determination part 102b may determine function influencing effect based on fitting with a pocket structure possibly relating to the functionality of the protein. To be more specific, if it is known that binding to a specific pocket structure results in manifestation of inhibitory effect, activating effect, etc., then the determination part 102b may evaluate the inhibitory effect or activating effect based on actual fitting with the pocket structure.

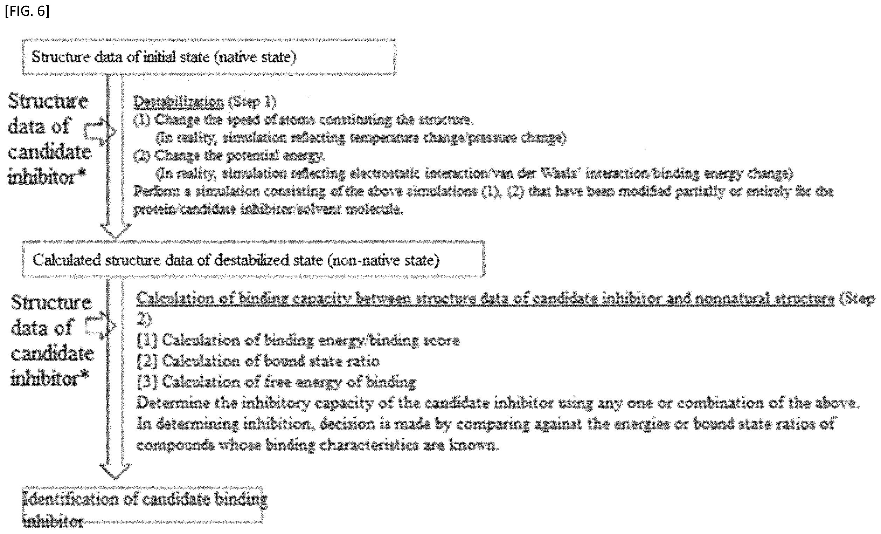

Now, FIG. 6 is flowchart showing an example of the selective functional substance search method using the destabilization part 102a and determination part 102b of the selective functional substance search system 100. It should be noted that, while the following explanation primarily uses inhibitory effect as an example of the function of the functional substance to search for, this has no limiting effect and the search for functional substance may be performed by evaluating such functions as accelerative effect, stabilizing effect, and activating effect. Specifically, while the following examples may be explained primarily by using search for inhibitor as an example, the examples may be similarly applied to search for such functional substances as accelerators, coagulants, stabilizers, and activators. Accordingly, in the embodiment (including the Examples), the terms "inhibitor" as well as "accelerator," "coagulant," "stabilizer," "activator," "functional substance," etc., may be applied interchangeably according to the purpose of each search target. As described above, once the destabilization part 102a simulates a non-native state representing a destabilized version of the native state (step 1), the determination part 102b calculates the binding capacity between the structure data of the candidate inhibitor and the non-native state (step 2). As examples of the calculation operation, the determination part 102b may use any one, or combination of a multiple, of (1) Calculation of binding energy/binding score, (2) Calculation of bound state ratio, and (3) Calculation of free energy of binding, to determine the inhibitory capacity of the candidate inhibitor. Then, the determination part 102b may decide the inhibitory effect by comparing the result against the energies or bound state ratios of compounds whose binding characteristics are known. It should be noted that, as described above, and as shown in the figures, the candidate inhibitor may be added before or after the protein is destabilizer in the simulation. Similarly, in the simulation, structure data of a protein substrate or binding substance, surfactant, hydrope, etc., may be added before or after the protein is destabilized. It should be noted that the determination result by the determination part 102b may be stored in an evaluation file 106b.

Meanwhile, the device control part 102c is a control means for controlling the input part 112 and output part 114, as well as an external device 200 and other devices. As an example, the device control part 102c may output the determination result from the determination part 102b to the output part 114 being a monitor, printer, etc., or transmit the determination result to the external device 200.

In addition to the above, the device control part 102c may also perform device controls to execute the destabilization/restabilization process and determination process in the embodiment. For example, the device control part 102c may perform controls to execute the destabilization/restabilization step and determination step in the embodiment, by controlling the input unit 112 comprising the destabilization means/restabilization means and determination means such as a real-time PCR unit, etc., as well as the output part 114, as described above. For example, the device control part 102c may perform controls so that the destabilization/restabilization step, determination step, etc., which are carried out automatically utilizing a real-time PCR unit, etc., will be executed only for those candidate inhibitors for which a determination result indicating high inhibitory effect was obtained by the aforementioned simulation involving the destabilization part 102a and determination part 102b.

The foregoing provided an example of the constitution of the selective functional substance search system 100 in the embodiment. It should be noted that the selective functional substance search system 100 may be connected to the external device 200 via a network 300, in which case the communication control interface part 104 performs communication controls between the selective functional substance search system 100 and the network 300 (or a router or other communication unit). Specifically, the communication interface part 104 has a function to communicate data with other terminals via a communication line. Also, the network 300 has a function to interconnect the selective functional substance search system 100 and the external device 200, and it may be the Internet, etc., for example.

Additionally, the external system 200 may also have a function, when interconnected to the selective functional substance search system 100 via the network 300, to provide external databases relating to structure data, various parameters, simulation result data and various other data, as well as programs, etc., for instructing an information processing unit connected to it to execute methods for three-dimensional structure calculations and other computations.

Here, the external device 200 may be constituted as a WEB server, ASP server, etc. Also, in terms of hardware configuration, the external device 200 may be configured using a generally and commercially available workstation, personal computer or other information processing unit and accessories thereto. Additionally, each function of the external device 200 is implemented by the CPU, disk unit, memory unit, input unit, output unit, communication control unit, etc., included in the hardware configuration of the external device 200, as well as by the programs, etc., controlling these units.

This completes the explanation of the constitution and processing examples of the embodiment.

### [Examples]

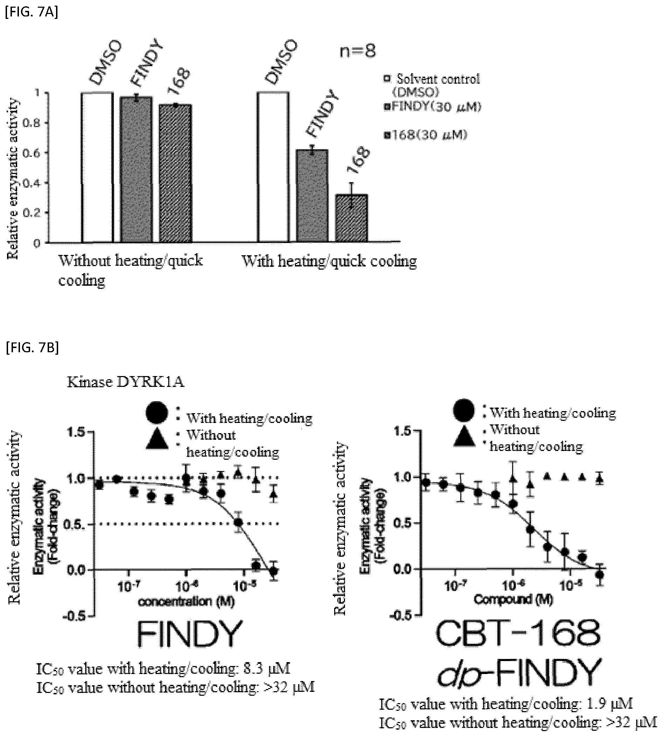

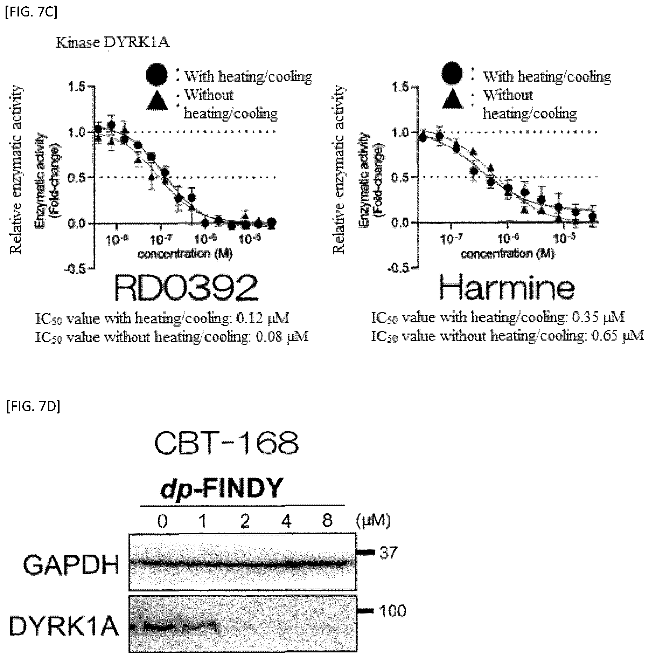

Various examples of embodiments pertaining to the present invention, in addition to the aforementioned one in FIG. 4, are explained below. Now, FIG. 7A is a figure showing the inhibitory effects of heating/quick cooling on complete enzymatic activity in two types of specific inhibitors with a folding intermediate. Also, FIG. 7B is a figure showing that the inhibitory effects of heating/quick cooling on complete enzymatic activity in two types of for the folding intermediate are concentration-dependent. Meanwhile, FIG. 7C is a figure showing the inhibitory effects measured using the known substance RD0392 and the known substance Harmine as controls. Additionally, FIG. 7D is a figure showing the test results in cultured cells. It should be noted that FINDY and compound 168 (also known as CBT-168/dp-FINDY) are expressed by the chemical formulas below, and have been confirmed, by the inventors of the invention under the present application for patent, to have effects as two types of specific inhibitors with the folding intermediate, as described above. It should be noted that Harmine is a plant-derived alkaloid compound, and because its inhibitory activity on the kinase DYRK1A is reported in a number of papers, it was used as a positive control under prior art.

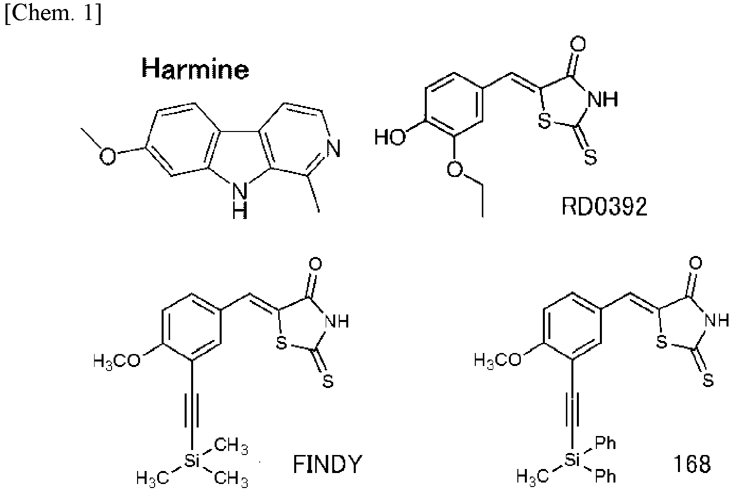

As shown in FIG. 7A, these two types of specific inhibitors FINDY and 168 (CBT-168/dp-FINDY) with the folding intermediate did not exhibit inhibitory effect on the complete protein that was not heated/cooled, but exhibited an inhibitory effect when heating/cooling, which embodies the destabilization/restabilization in the embodiment, was performed. This confirms that the destabilization/restabilization in the embodiment reproduced a folding intermediate structure. Also, as shown in FIG. 7B, these two types of specific inhibitors FINDY and 168 (CBT-168/dp-FINDY) with the folding intermediate were confirmed to exhibit an inhibitory effect in a concentration-dependent manner. It should be noted that, as shown by the control test in FIG. 7C, RD0392 and Harmine known to have inhibitory activity on the kinase DYRK1A exhibited an inhibitory effect also on the complete protein that was not heated/cooled, and unlike in the embodiment (FIG. 7A, FIG. 7B, etc.), they did not exhibit a specific inhibitory effect with the folding intermediate undergoing heating/cooling for destabilization/restabilization.

Also, compound 168 (also known as CBT-168/dp-FINDY) was confirmed to exhibit an inhibitory effect also in cultured cells. To be more specific, a study was conducted by western blotting using the cultured cell line HEK293. Specifically, the HEK293 cell line was cultured for 4 days in the presence of compound 168 (dp-FINDY), after which a cell extract liquid was prepared. Using the SDS-PAGE and western blotting methods, they were detected using antibodies specific thereto, respectively. As a result, the DYRK1A band became fainter in a manner dependent on the additive amount of dp-FINDY, as shown in FIG. 7D, confirming that dp-FINDY had an activity to decompose /remove the target kinase DYRK1A in the cultured cells.

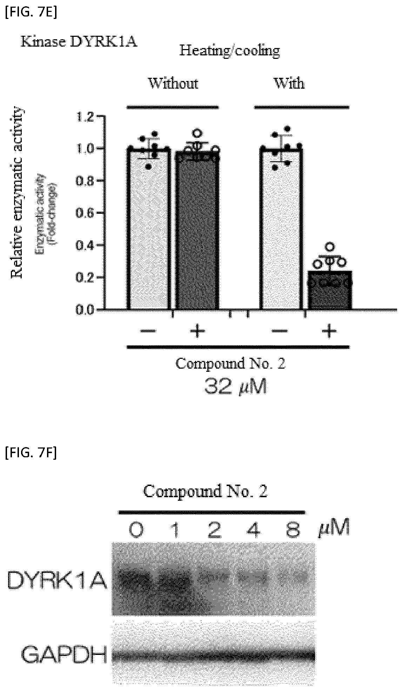

Also, as a result of additional studies conducted in relation to the above test, compound No. 2 expressed by the chemical formula below was discovered as an inhibitor pertaining to the embodiment. FIG. 7E is a figure showing the inhibitory effect of specific inhibitor compound No. 2 with the folding intermediate under heating/quick cooling on complete enzymatic activity, while FIG. 7F is a figure showing the western blotting detection results with compound No. 2. It should be noted that compound No. 2 is a structural analogue to green tea catechin EGCG. (Reference: Refer to compound 5 in the following papers: https://pubmed.ncbi.nlm.nih.gov/20596600/, https://pubmed.ncbi.nlm.nih.gov/20045338/).

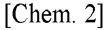

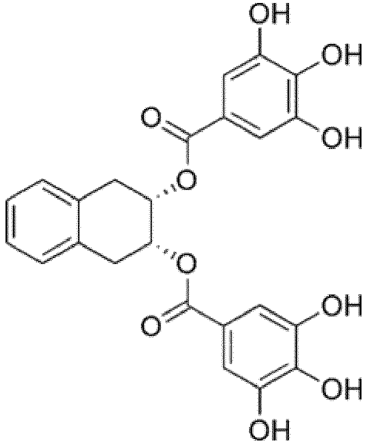

As shown in FIG. 7E, the newly discovered compound No. 2 exhibited a specific inhibitory effect only with the folding intermediate undergoing heating/cooling for destabilization/restabilization. Meanwhile, a western blotting detection using the cultured cell line HEK293 was also conducted. Specifically, the HEK293 cell line was cultured for 4 days in the presence of compound No. 2, after which a cell extract liquid was prepared. Using the SDS-PAGE and western blotting methods, DYRK1A and GAPDH were detected using antibodies specific to the two. As a result, the DYRK1A band became fainter in a manner dependent on the additive amount of compound No. 2, as shown in FIG. 7F, confirming that compound No. 2 had an activity to decompose /remove the target kinase DYRK1A in the cultured cells.

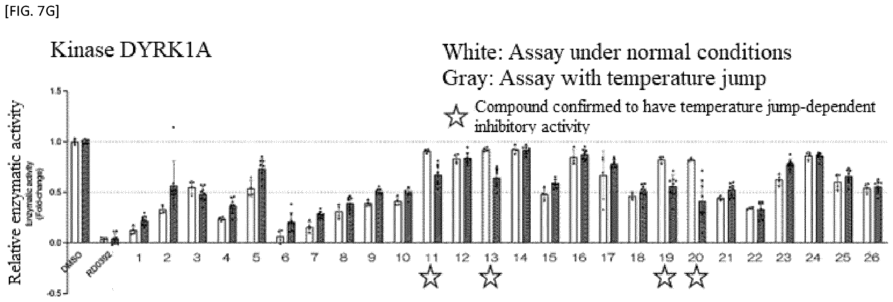

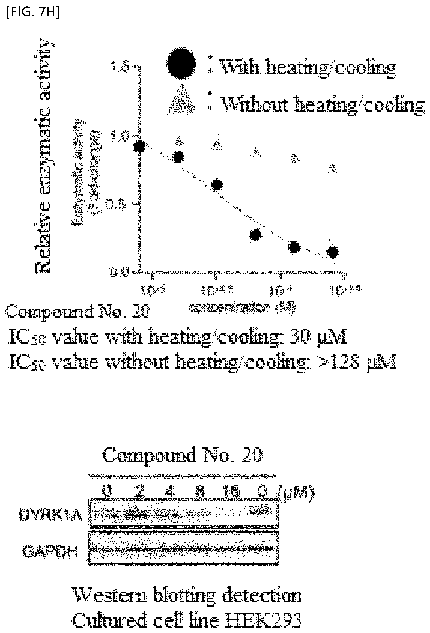

Also, a screening was conducted by expanding the above Examples with the aim of searching for inhibitors and activators specific to the folding intermediate in the embodiment. FIG. 7G is a figure showing the small-scale inhibitor/activator screening results with respect to the kinase DYRK1A. This library is a small-scale structural analogue library (Nos. 1 to 26) owned by the research lab of the inventors of the invention under the present application for patent. FIG. 7H is a figure showing the inhibitory effect of compound No. 20 identified as an inhibitor in the embodiment as a result of screening.

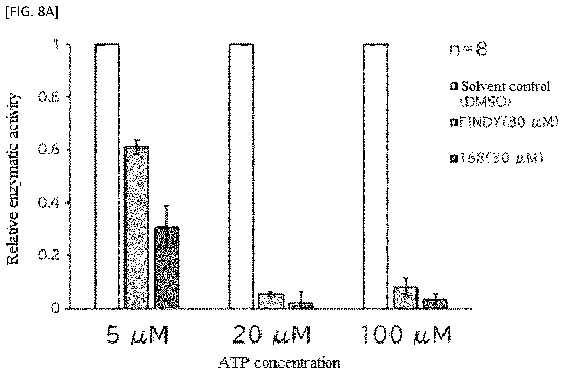

As shown in FIG. 7G, compound No. 11, No. 13, No. 19, and No. 20 were identified, in the small-scale library of 26 types of structural analogues, as compounds having an inhibitory activity specific to the folding intermediate undergoing heating/cooling (specifically temperature jump) for destabilization/restabilization. Also, as shown in FIG. 7H, compound No. 20 was confirmed to have a concentration-dependent inhibitory effect. It should be noted that, as shown in the bottom figure in FIG. 7H, a western blotting detection was conducted in the cultured cell line HEK293 in the same manner as described above. As a result, the DYRK1A band disappeared when compound No. 20 was added, as shown in the bottom figure in FIG. 7H, confirming that it had an activity to decompose/remove the target kinase DYRK1A in the cultured cells. Now, FIG. 8A is a graph showing the relationship of ATP concentration and inhibitory effect.

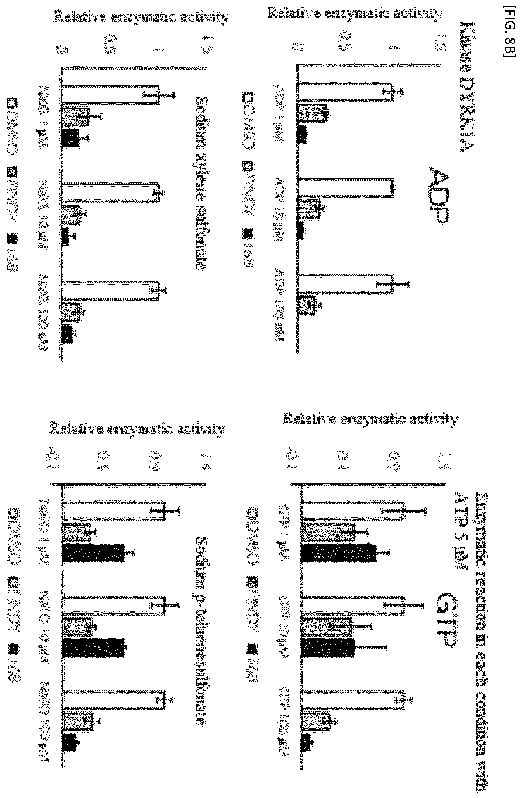

As shown in FIG. 8A, it was confirmed that the inhibitory effects of the two types of specific inhibitors FINDY and 168 (CBT-168/dp-FINDY) with the folding intermediate would improve further in the presence of highly concentrated ATP. Since ATP is a type of hydrotrope, this suggests a possibility that presence of a hydrotrope may help stabilize a complex consisting of an inhibitor and a non-native state (intermediate). Now, FIG. 8B is a graph showing the relationships between concentrations of ADP, GDP, sodium xylene sulfonate and sodium p-toluenesulfonate, and inhibitory effects.

As shown in FIG. 8B, it was confirmed that the inhibitory effects of the two types of specific inhibitors FINDY and 168 (CBT-168/dp-FINDY) with the folding intermediate would improve in the presence of ADP, GDP, sodium xylene sulfonate, and sodium p-toluenesulfonate. This suggests that, as hydrotropes, these ADP, GDP, sodium xylene sulfonate, and sodium p-toluenesulfonate may also help stabilize a complex consisting of an inhibitor and a non-native state (intermediate).

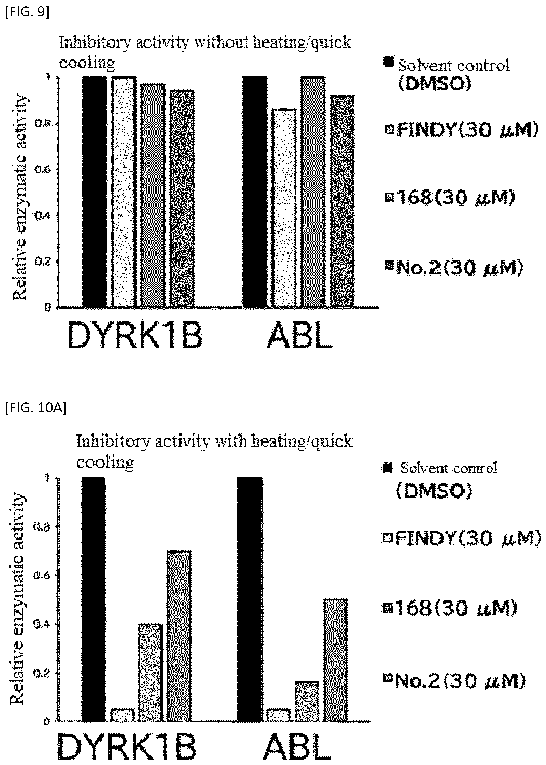

Now, FIG. 9 is a figure showing the inhibitory activities without heating/cooling attributable to three types of specific inhibitors FINDY, 168 and No. 2 with the folding intermediate, while FIG. 10A is a figure showing the inhibitory activities with heating/cooling attributable to three types of specific inhibitors FINDY, 168, and No. 2 with the folding intermediate.

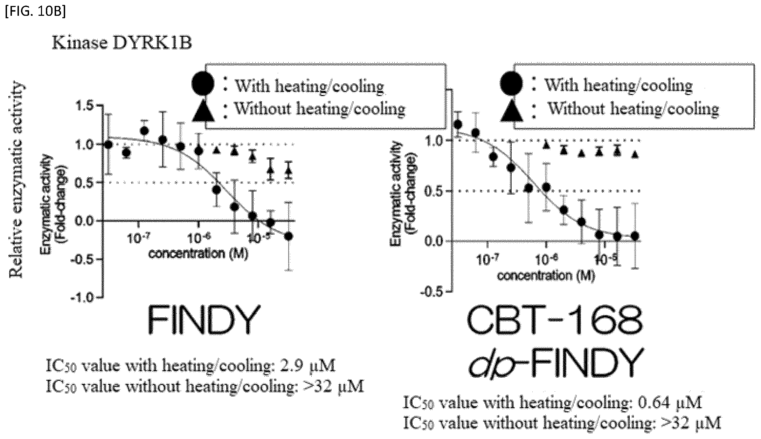

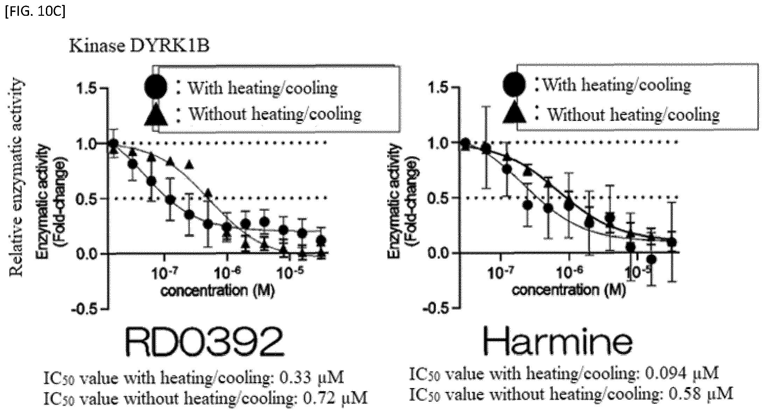

As shown in FIG. 9, while the specific inhibitors with the folding intermediate did not exhibit inhibitory activity without heating/cooling, they remarkably exhibited inhibitory activity when the heating/quick cooling in the embodiment was performed (FIG. 10A). This confirms that the inhibitors selectively exhibit an inhibitory effect on the protein structure that was destabilized according to the embodiment. Now, FIG. 10B is a figure showing that the inhibitory effects of two types of specific inhibitors with the folding intermediate under heating/quick cooling on complete enzymatic activity in are concentration-dependent. Also, FIG. 10C is a figure showing the inhibitory effects measured using the known substance RD0392 and the known substance Harmine as controls.

As shown in FIG. 10B, these two types of specific inhibitors FINDY and 168 (CBT-168/dp-FINDY) with the folding intermediate were confirmed to exhibit an inhibitory effect on the kinase DYRK1B in a concentration-dependent manner. It should be noted that, as shown by the control test in FIG. 10C, RD0392 and Harmine exhibited an inhibitory effect on the kinase DYRK1B, also with the complete protein that was not heated/cooled, and unlike in the embodiment (FIG. 10A, FIG. 10B, etc.), they did not exhibit a specific inhibitory effect exclusively with the folding intermediate undergoing heating/cooling for destabilization/restabilization.

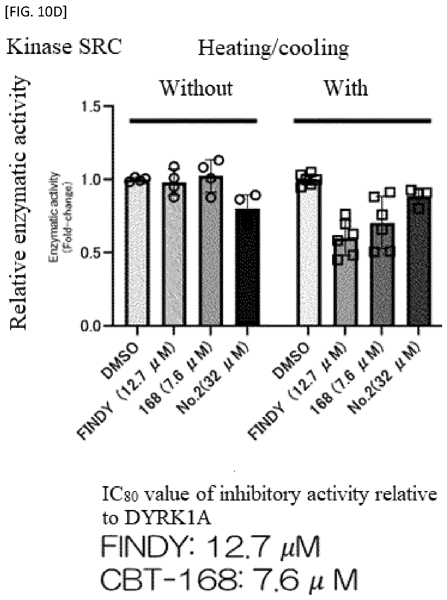

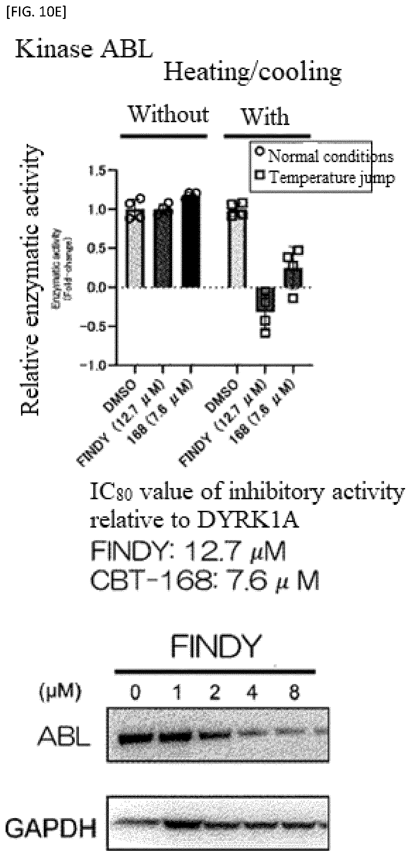

Here, inhibitory effects on other kinases were studied. FIG. 10D is a figure showing the inhibitory activities of three types of specific inhibitors FINDY, 168, and No. 2 with the folding intermediate, with and without heating/cooling, on the kinase SRC. It should be noted that, for FINDY and CBT-168, the IC₈₀ concentration for temperature-jump inhibition of DYRK1A was used. Also, FIG. 10E is a figure showing the inhibitory activities of three types of specific inhibitors FINDY, 168, and No. 2 with the folding intermediate, with and without heating/cooling, on the kinase ABL.

As a result, FINDY and CBT-168 inhibited the kinase SRC in a temperature jump-dependent manner, as shown in FIG. 10D. However, these inhibitory activities were weaker than those on DYRK1A. As stated, selectivity of the three types of specific inhibitors with the folding intermediate was possible to compare between the kinases. Also, as shown in FIG. 10E, FINDY strongly inhibited the kinase ABL in a temperature jump-dependent manner. This inhibitory activity was stronger than that on DYRK1A. Additionally, a western blotting detection using the cultured cell line HEK293 was conducted. Specifically, the HEK293 cell line was cultured for 4 days in the presence of FINDY, after which a cell extract liquid was prepared. Using the SDS-PAGE and western blotting methods, ABL and GAPDH were detected using antibodies specific to the two. As a result, the ABL band became fainter in a manner dependent on the additive amount of FINDY, as shown in the bottom figure in FIG. 10E. This confirmed FINDY to have an activity to decompose/remove the target kinase ABL in the cultured cells.

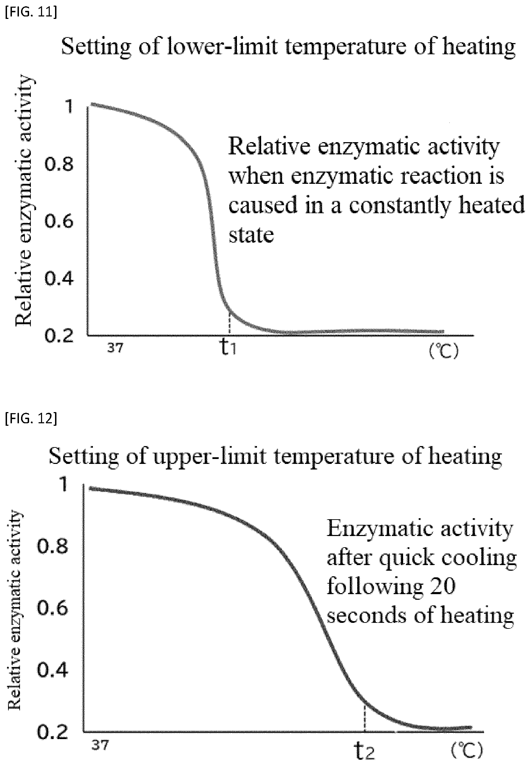

Next, the results of studying the temperature conditions for heating/quick cooling in the embodiment are presented. As shown in FIG. 11, the temperature *t*1 at which the enzymatic activity of the protein was lost in a constantly heated state, was adopted as the lower-limit value of heating. Also, as shown in FIG. 12, the temperature t2 at which the enzymatic activity was lost even when quick cooling was performed after heating for a relatively short period of 20 seconds, was adopted as the upper-limit value of heating.

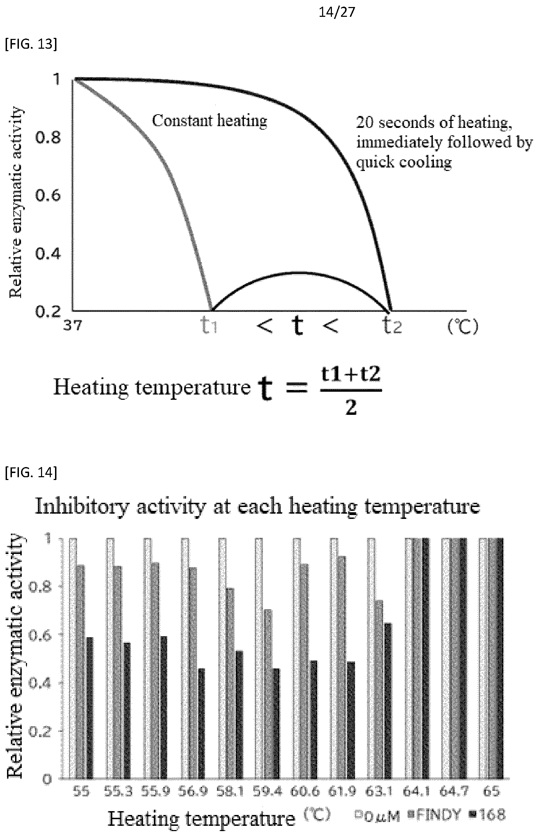

Specifically, as shown in FIG. 13, the temperature *t* between these temperature *t*1 at which deactivation occurs after a long period and temperature *t*2 at which deactivation occurs even after a short period of heating followed by quick cooling (*t*1<*t*<*t*2) provides for the condition for the maximum range of temperature change in the embodiment. Accordingly, it is estimated that preferably heating temperature *t* = (*t*1+*t*2*)*/2*.*

Therefore, the following test was conducted to study more appropriate temperature conditions among those satisfying this temperature *t* (*t*1<*t*<*t*2)*.*

### [Test Conditions]

Kinase DYRK1A protein mass: 25 ng
DMSO concentration: 0.3% in 25 µL
ATP/substrate peptide (DYRKtide peptide: amino-acid sequence RRRFRPASPLRGPPK) concentration: 5 µM each
Volume at enzymatic reaction: 25 µL
Volume in thermal cycler (Biometra TAdvanced Model 96SG): 15 µL
Enzymatic reaction period: 2 hrs. 30 min.
Heating period: 20 sec.
Rate of heating: 8°C/sec.
Heating temperature: 37°C to 65°C
Inhibitors: FINDY/compound 168, 30 µM each

### [Test Procedure]

1. The DYRK1A protein, inhibitors (solvent DMSO alone was added in the case of "Inhibitor 0 µM"), buffer (final concentrations: 5 mM MOPS pH7.2, 2.5 mM beta-glycerol-phosphate, 5 mM MgCl₂, 1 mM EGTA, 0.4 mM EDTA, 0.05 mM DTT) and ultrapure water were mixed in tubes placed in a white 96-well PCR plate. [Volume: 15 µL, DYRK1A protein mass: 25 ng, inhibitor concentration: 30 µM]
2. The plate was set in a thermal cycler and heated for 20 seconds at each heating temperature, and then cooled for 10 seconds at 3°C. [Rate of heating/rate of cooling both 8°C/sec.]
3. Upon completion of cooling, it was removed immediately and 10 µL of ATP/substrate peptide mixture solution was added. [Volume: 15 µL → 25 µL, ATP/substrate peptide concentration 5 µM]
4. It was set in the thermal cycler again to cause enzymatic reaction for 2 hours 30 minutes at 20°C.
5. It was picked up from the thermal cycler and 25 µL of Kinase Glo reaction solution was added, and from the resulting luminescence value, the remaining amount of ATP was measured to evaluate the enzymatic activity. [Volume: 25 µL → 50 µL]

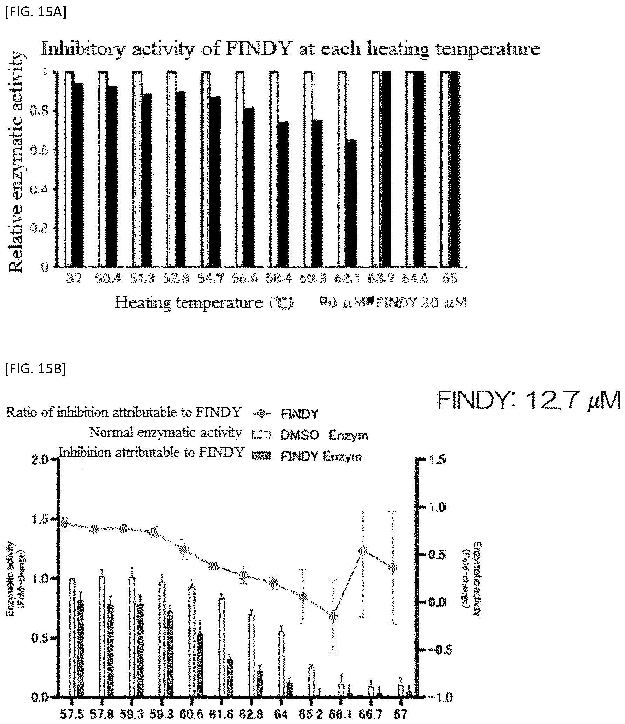

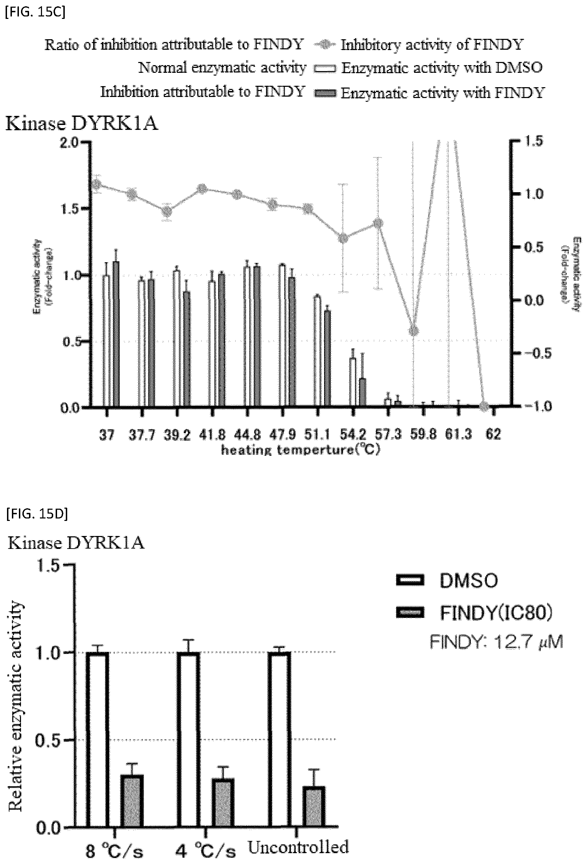

Consequently, the results shown in Table 1 (graph in FIG. 14) and Table 2 (graph in FIG. 15) below were obtained. The numerical values, each representing an average of n = 2 results, did not have variation.

**[Table 1]**

| | Heating temperature (°C) | 55 | 55.3 | 55.9 | 56.9 | 58.1 | 59.4 |
|---|---|---|---|---|---|---|---|
| Negative control | Luminescence value | 3473576 | 3258472 | 3424368 | 3310504 | 3609424 | 3523608 |
| Inhibitor 0 µM | Luminescence value | 388336 | 458508 | 594344 | 746712 | 1201996 | 1406948 |
| | Enzymatic activity | 3085240 | 2799964 | 2830024 | 2563792 | 2407428 | 2116660 |
| FINDY 30 µM | Luminescence value | 741900 | 783908 | 894988 | 1064500 | 1702208 | 2032152 |
| | Enzymatic activity | 2731676 | 2474564 | 2529380 | 2246004 | 1907216 | 1491456 |
| | Relative enzymatic activity | 0.88540146 | 0.88378422 | 0.893766272 | 0.876047667 | 0.792221408 | 0.704627101 |
| 168 30 µM | Luminescence value | 1656144 | 1673544 | 1752288 | 2133624 | 2331480 | 2557380 |
| | Enzymatic activity | 1817432 | 1584928 | 1672080 | 1176880 | 1277944 | 966228 |
| | Relative enzymatic activity | 0.589073135 | 0.566052992 | 0.590835979 | 0.459038799 | 0.530833736 | 0.456487107 |

| | Heating temperature (°C) | 606 | 61.9 | 63.1 | 64.1 | 64.7 | 65 |
|---|---|---|---|---|---|---|---|
| Negative control | Luminescence value | 3397480 | 3465520 | 3591000 | 3429712 | 3639288 | 3766000 |
| Inhibitor 0 µM | Luminescence value | 2029496 | 2376184 | 2826296 | 3078656 | 3302344 | 3284772 |
| | Enzymatic activity | 1367984 | 1089336 | 764704 | 351056 | 336944 | 481228 |
| FINDY 30 µM | Luminescence value | 2177084 | 2459820 | 3025240 | 2971552 | 3085788 | 3160392 |
| | Enzymatic activity | 1220396 | 1005700 | 565760 | 458160 | 553500 | 605608 |
| | Relative enzymatic activity | 0.892112773 | 0.923222954 | 0.739841821 | 1.305090926 | 1.642706206 | 1.258463764 |
| 168 30 µM | Luminescence value | 2723816 | 2937944 | 3096488 | 2998376 | 3244648 | 3252544 |
| | Enzymatic activity | 673664 | 527576 | 494512 | 431336 | 394640 | 513456 |
| | Relative enzymatic activity | 0.492450204 | 0.484309708 | 0.64667113 | 1.228681464 | 1.171233202 | 1.066970334 |

**[Table 2]**

| | Heating temperature (°C) | 37 | 50.4 | 51.3 | 52.8 | 54.7 | 56.6 |
|---|---|---|---|---|---|---|---|
| Negative control | Luminescence value | 3701640 | 3484836 | 3520892 | 3456272 | 3455484 | 3545532 |
| Inhibitor 0 µM | Luminescence value | 60832 | 46748 | 71912 | 101356 | 167772 | 384836 |
| | Enzymatic activity | 3640808 | 3438088 | 3448980 | 3354916 | 3287712 | 3160696 |
| FINDY 30 µM | Luminescence value | 280968 | 301424 | 467664 | 452944 | 583992 | 974436 |
| | Enzymatic activity | 3420672 | 3183412 | 3053228 | 3003328 | 2871492 | 2571096 |
| | Relative enzymatic activity | 0.939536498 | 0.925925107 | 0.885255351 | 0.895202145 | 0.87340132 | 0813458808 |

| | Heating temperature (°C) | 58.4 | 60.3 | 62.1 | 63.7 | 64.6 | 65 |
|---|---|---|---|---|---|---|---|
| Negative control | Luminescence value | 3663268 | 3524024 | 3530292 | 3482908 | 3518472 | 3450080 |
| Inhibitor 0 µM | Luminescence value | 643492 | 1343652 | 2175380 | 2870472 | 3138172 | 3189232 |
| | Enzymatic activity | 3019776 | 2180372 | 1354912 | 612436 | 380300 | 260848 |
| FINDY 30 µM | Luminescence value | 1437480 | 1881724 | 2659332 | 2833960 | 3132768 | 3055024 |
| | Enzymatic activity | 2225788 | 1642300 | 870960 | 648948 | 385704 | 395056 |
| | Relative enzymatic activity | 0.737070564 | 0.753220093 | 0.642816655 | 1.059617658 | 1.014209834 | 1.51406533 |

As shown in FIG. 14 and FIG. 15A, FINDY exhibited a stronger inhibitory effect of 0.7 or lower in relative enzymatic activity at around 58 to 62 or 63°C within the test temperature conditions of 55°C to 65°C. Also, compound 168 exhibited a stronger inhibitory effect of 0.7 or lower in relative enzymatic activity at around 55°C to 63°C. This implies that, when targeting an enzyme protein for which a suitable temperature is around 37°C, the search for an inhibitor that selectively binds to the protein's non-native state and inhibits its function is facilitated under heating conditions of around 55°C to 63°C.

It should be noted that FIG. 15B presents the data in FIG. 14 and FIG. 15A in an easier-to-understand manner. It should be noted that, for the compound concentrations, the IC₈₀ concentration with temperature jump was used. Here, "IC₈₀" refers to a concentration at which 80% of enzymatic activity is inhibited (20% of activity remains). As shown in FIG. 15B, clearly the kinase DYRK1A was not inhibited at low heating temperatures. Also, it was found that high temperatures led to greater data variations. In other words, according to FIG. 15B, which shows the relative enzymatic activity under each condition based on the enzymatic activity at 57.5°C/DMSO being 1.0, the inhibitory activity attributable to FINDY was weak below 60°C. The inhibitory activity attributable to FINDY, relative to DMSO, was detected strongly until 64°C. On the other hand, the enzymatic activity dropped significantly above 65.2°C, resulting in data variation. The foregoing confirms that there is an optimum temperature.

Here, a study was conducted regarding the need for a cooling step as a reconstitution step in the embodiment. FIG. 15C is a figure showing the relative enzymatic activities, under heating at a constant temperature (no cooling step), with respect to the kinase DYRK1A. For the compound concentrations, the IC₈₀ concentration with temperature jump was used. As shown in FIG. 15C, inhibitory activity of approx. 20% to 30% was observed at 54.2°C or more. However, because the compound concentrations were such that 80% inhibition would be achieved with an added cooling step, it was confirmed that sufficient inhibitory activity could not be detected in this test where no cooling step was provided.

Also, a study was conducted regarding the rate of cooling in the embodiment. FIG. 15D is a figure showing the inhibitory effects, at varying rates of cooling, with respect to the kinase DYRK1A. For other than this test, cooling was performed at 8°C/sec. "Uncontrolled" indicates a setup where the 96-well plate was removed from the heating block and cooled naturally at room temperature. As shown in FIG. 15D, the rate of cooling did not affect the result at levels as low as natural cooling at room temperature. Although what would happen under slower cooling than under these conditions is unknown, it is expected that the loss of enzymatic activity will increase.

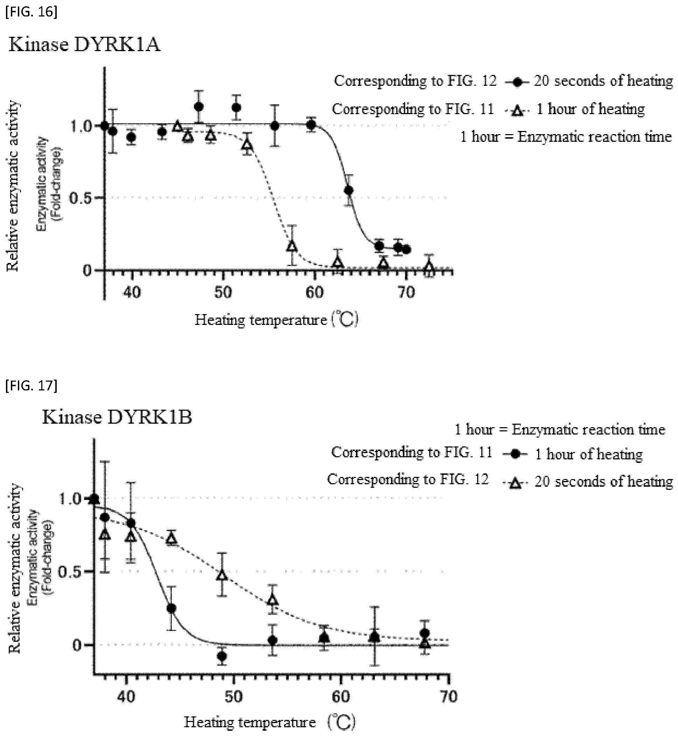

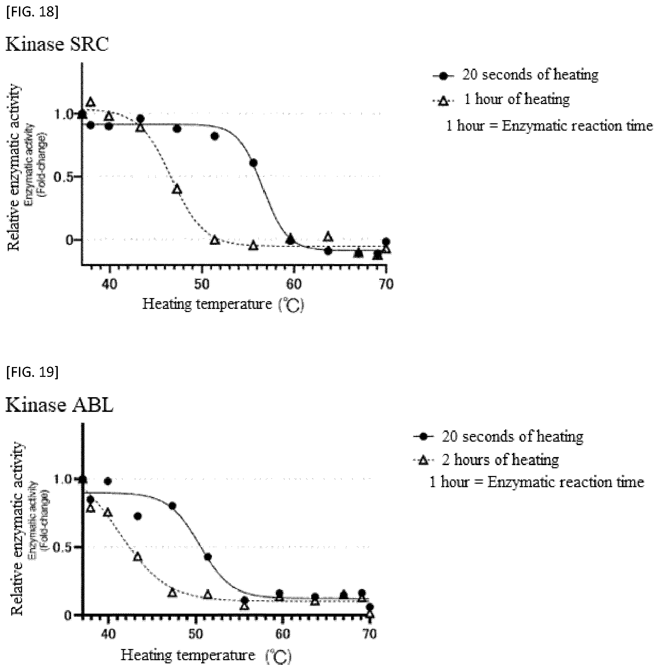

Here, additional studies were conducted regarding the kinases DYRK1A, DYRK1A, SRC and ABL, respectively, as a way to set the upper-limit temperatures and lower-limit temperatures in FIG. 11 to FIG. 13. FIG. 16 is a figure showing the study results on upper-limit temperature and lower-limit temperature for the kinase DYRK1A, FIG. 17 is a figure showing the study results on upper-limit temperature and lower-limit temperature for the kinase DYRK1B, FIG. 18 is a figure showing the study results on upper-limit temperature and lower-limit temperature for the kinase SRC, and FIG. 19 is a figure showing the study results on upper-limit temperature and lower-limit temperature for the kinase ABL. It should be noted that the relative enzymatic activity was calculated based on the enzymatic activity at 37°C being 1.0.

As a result, for the kinase DYRK1A, the upper-limit temperature of 67.0°C and lower-limit temperature of 57.5°C were calculated as temperature conditions based on the measured data, as shown in FIG. 16. Consequently, the setpoint heating temperature for the temperature jump test was set to 62.0°C. Also, for the kinase DYRK1B, the upper-limit temperature of 58.4°C and lower-limit temperature of 48.9°C were calculated as temperature conditions based on the measured data, as shown in FIG. 17. Consequently, the setpoint heating temperature for the temperature jump test was set to 53.7°C. Also, for the kinase SRC, the upper-limit temperature of 59.6°C and lower-limit temperature of 51.4°C were calculated as temperature conditions based on the measured data, as shown in FIG. 18. Consequently, the setpoint heating temperature for the temperature jump test was set to 55.5°C. Also, for the kinase ABL, the upper-limit temperature of 55.6°C and lower-limit temperature of 47.3 °C were calculated as temperature conditions based on the measured data, as shown in FIG. 19. Consequently, the setpoint heating temperature for the temperature jump test was set to 52.0°C.

### [Examples Other Than on Kinases]

Next, inhibitors and activators were searched for and studied with respect to the monoamine oxidase MAO-A and protease Calpain-I as proteins other than the aforementioned kinases.

It should be noted that the plant-derived compound library (1 to 34) used in the test contains flavonol/catechin compounds commercially available from the following companies (FUJIFILM Wako Pure Chemical Corporation, Tokyo Chemical Industry Co., Ltd., Merck & Co., Inc., and Nacalai Tesque, Inc.).

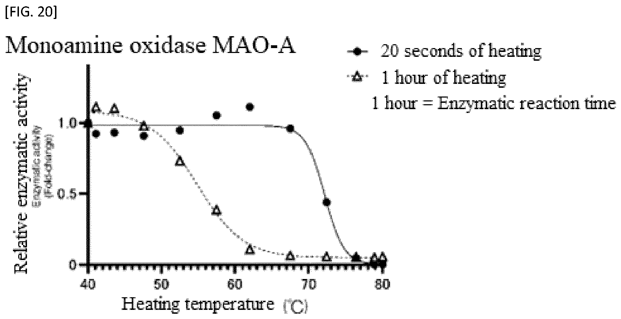

Also, the monoamine oxidase MAO-A was studied as a way to set the upper-limit temperatures and lower-limit temperatures in FIG. 11 to FIG. 13. FIG. 20 is a figure showing the study results on upper-limit temperature and lower-limit temperature for the monoamine oxidase MAO-A. It should be noted that the test was conducted according to the product manual for Promega's MAO-A-Glo^{™} Assay Systems. It should be noted that the relative enzymatic activity was calculated based on the enzymatic activity at 40°C being 1.0.

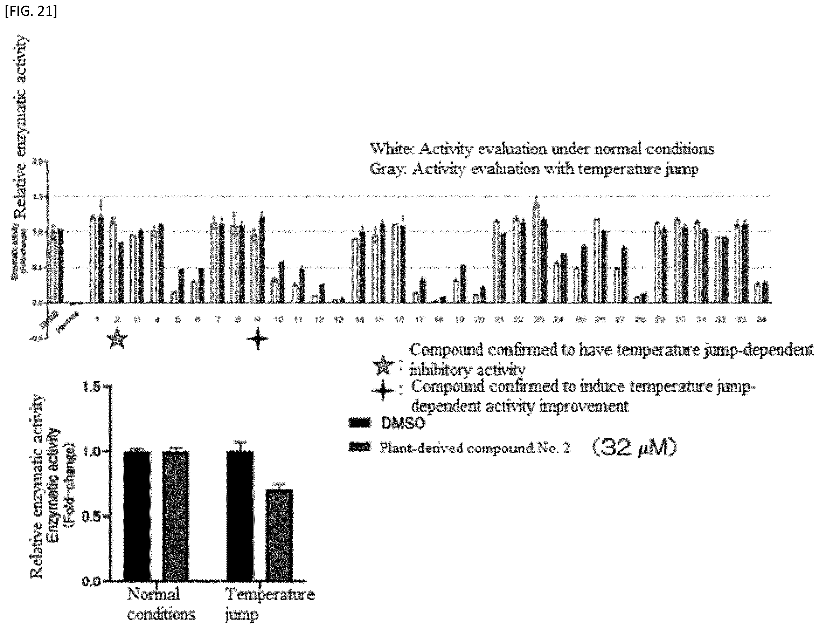

As a result, for the kinase MAO-A, the upper-limit temperature of 76.4°C and lower-limit temperature of 62.0°C were calculated as temperature conditions based on the measured data, as shown in FIG. 20. Consequently, the setpoint heating temperature for the temperature jump test was set to 69.2°C. FIG. 21 is a figure showing the small-scale inhibitor screening results with respect to the monoamine oxidase MAO-A.

As a result of screening, a temperature jump-dependent inhibitory activity was confirmed with compound No. 2, as shown in FIG. 21 (more detailed results are shown in the bottom figure in FIG. 21). Also, a temperature jump-dependent activity improvement was confirmed with compound No. 9.

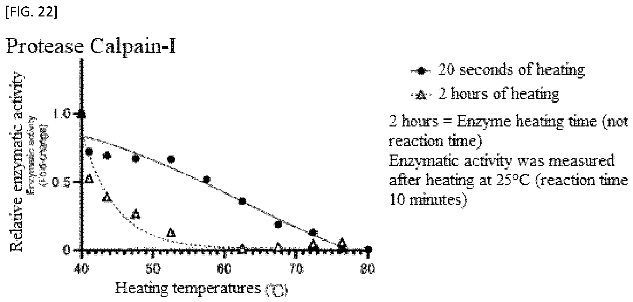

Also, the protease Calpain-I was studied as a way to set the upper-limit temperatures and lower-limit temperatures in FIG. 11 to FIG. 13. FIG. 22 is a figure showing the study results on upper-limit temperature and lower-limit temperature for the protease Calpain-I. It should be noted that the test was conducted according to the product manual for Promega's Calpain-Glo^{™} Assay Systems. It should be noted that the relative enzymatic activity was calculated based on the enzymatic activity at 40°C being 1.0.

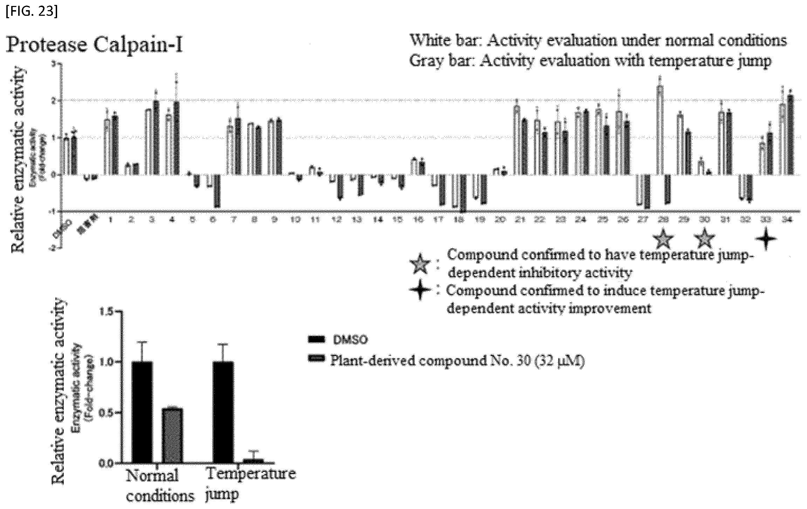

As a result, for the protease Calpain-I, the upper-limit temperature of 76.4°C and lower-limit temperature of 57.5°C were calculated as temperature conditions based on the measured data, as shown in FIG. 22. Consequently, the setpoint heating temperature for the temperature jump test was set to 60.0°C. It should be noted that this temperature is not the median value between the upper limit and the lower limit. Given the excessive difference between the upper limit and the lower limit, the temperature was set to around where the activity would become one half that at the upper-limit temperature setting. FIG. 23 is a figure showing the small-scale inhibitor screening results with respect to the protease Calpain-I.

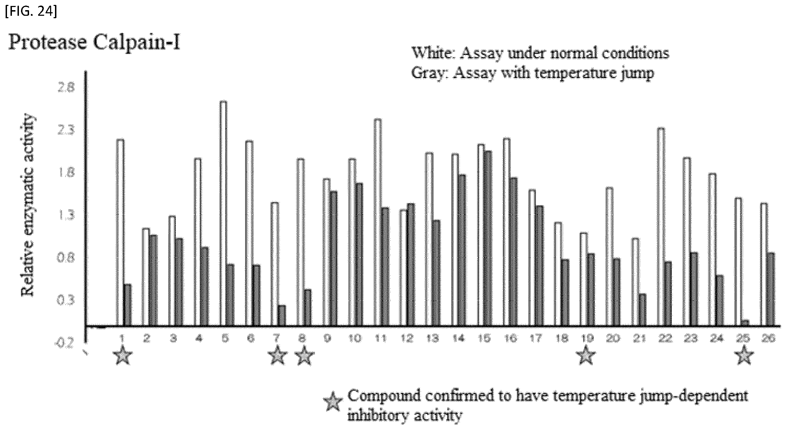

As a result of screening, a temperature jump-dependent inhibitory activity was confirmed with compounds No. 28 and No. 30, as shown in FIG. 23 (more detailed results on compound No. 30 are shown in the bottom figure in FIG. 23). Also, a temperature jump-dependent activity improvement was confirmed with compound No. 33. Meanwhile, FIG. 24 is a figure showing the screening results pertaining to a separate small-scale structural analogue library (Nos. 1 to 26).

As shown in FIG. 24, a temperature jump-dependent inhibitory activity was confirmed with compounds No. 1, No. 7, No. 8, No. 19, and No. 25 in this library.

### [Examples in Silico]

Examples of molecular dynamics (MD) simulations with temperature jump are explained below.

It should be noted that, in the MD simulations, a calculation period of 100 ns was used as the condition for how many times the heating/cooling would be repeated. Specifically, cooling was performed for 10 ns and heating, for 10 ns, for a total of 20 ns, and this was repeated five times.

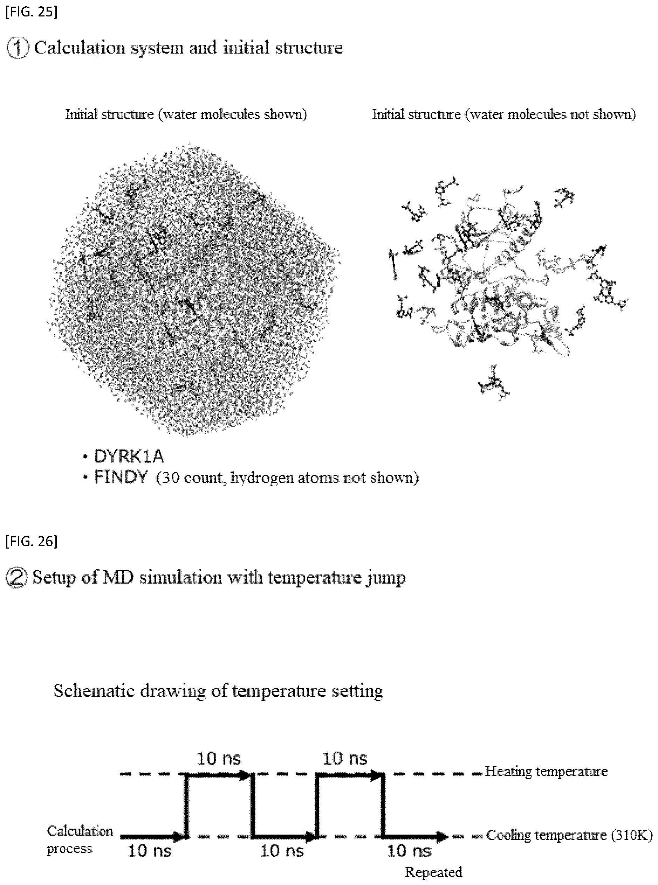

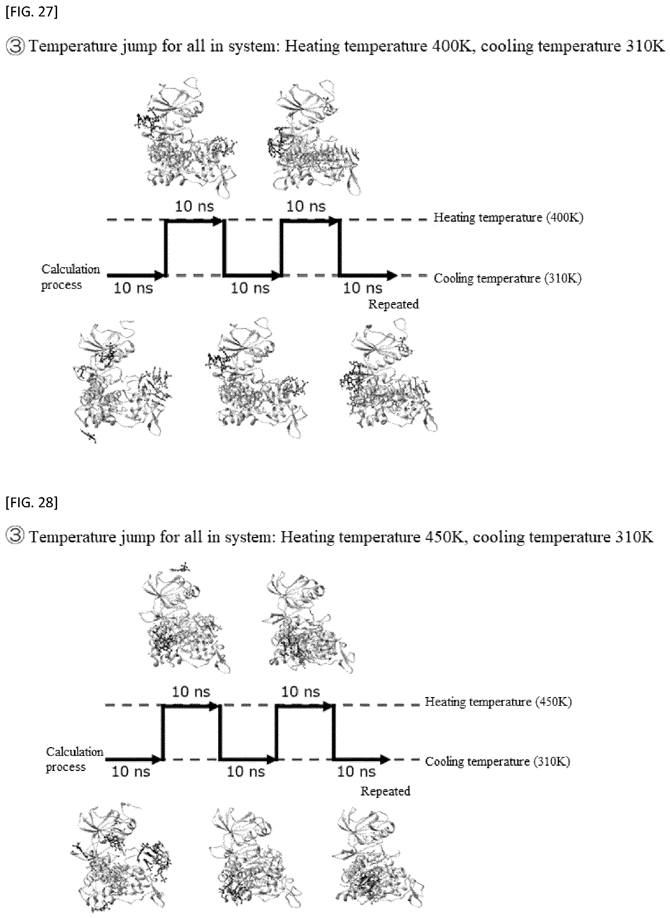

Now, FIG. 25 is a figure showing (1), "Calculation system and initial structure of DYRK1A-FINDY." Also, FIG. 26 is a schematic drawing of the temperature settings in (2), "Setup of MD simulation with temperature jump." FIG. 27 to FIG. 31 are figures showing the simulation results with each heating temperature set to 400K, 450K, 500K, 550K, and 600K, respectively.

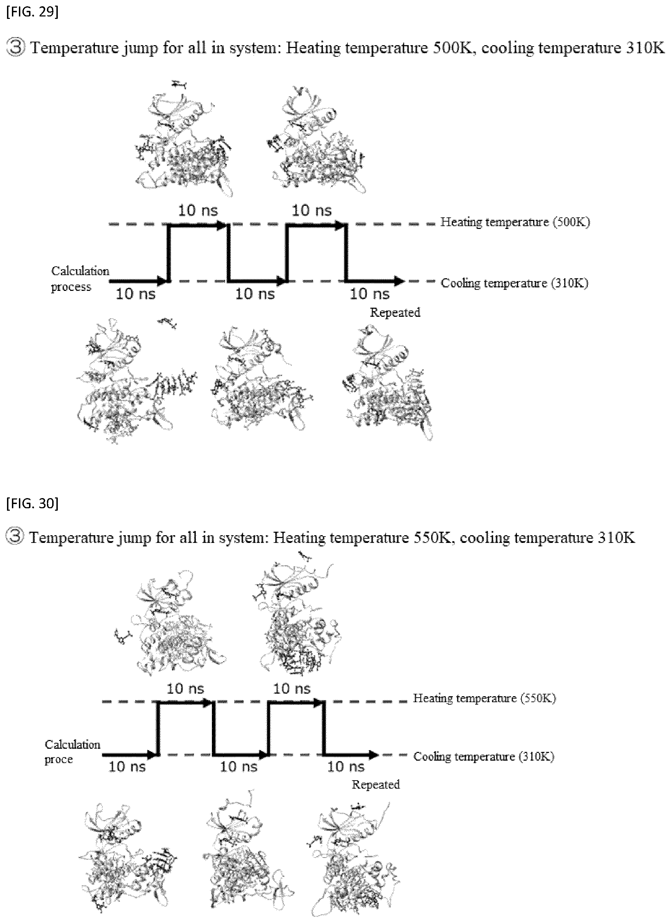

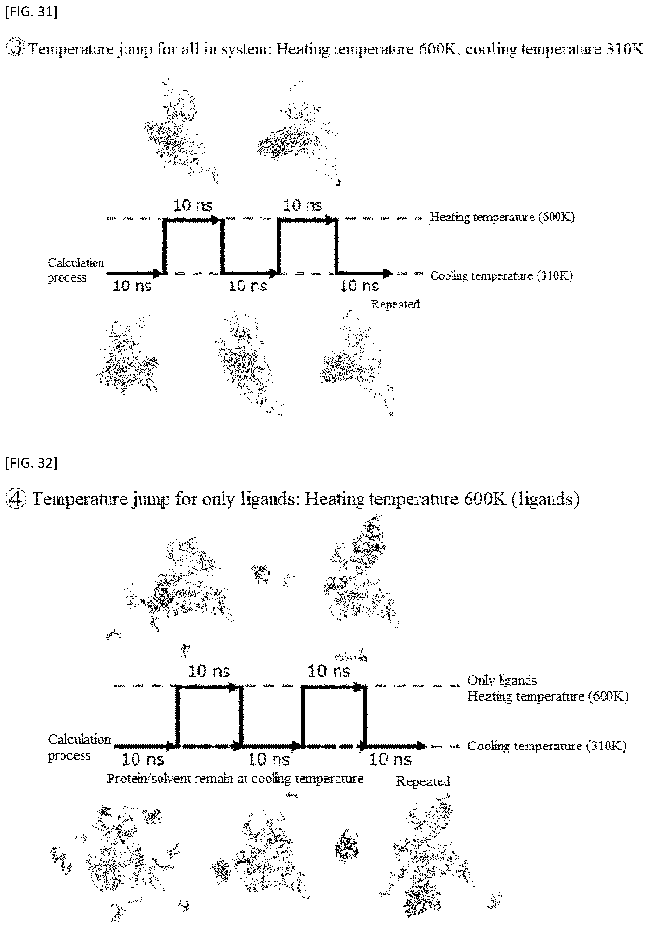

As shown in FIG. 27 to FIG. 31, while the protein structure was retained at the heating temperatures of 400K (FIG. 27), 450K (FIG. 28), and 500K (FIG. 29), the structure partially came loose when 550K (FIG. 30) and 600K (FIG. 31) were reached. As a result, heating temperatures of 450 to 600K were considered favorable when jumping the temperature for all in the system.

Also, indirectly heating the surface of the protein by not heating all in the system, but by jumping the temperature for only ligands, was studied. FIG. 32 to FIG. 34 are figures showing the simulation results with each heating temperature set to 600K, 800K, and 1000K, respectively, for only ligands.

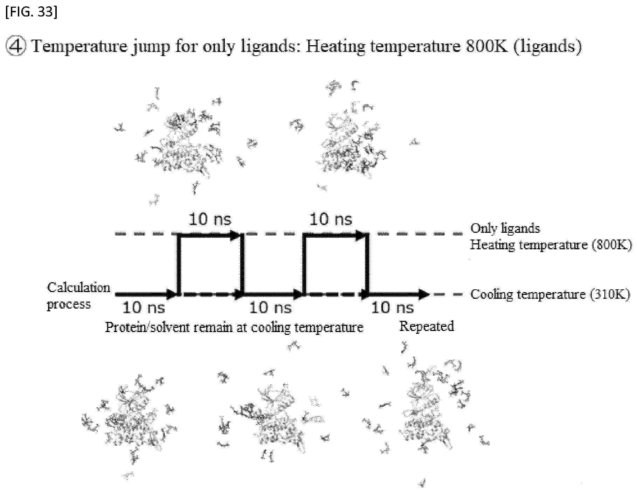

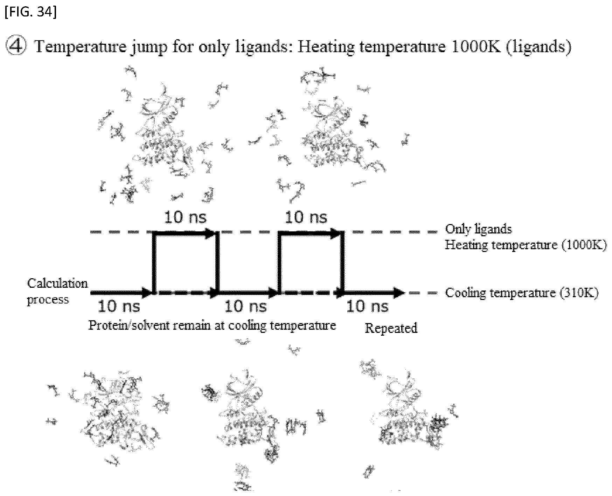

As a result, it was found that the structure of the protein was retained in every case at 600K (FIG. 32), 800K (FIG. 33), or 1000K (FIG. 34). Accordingly, ligand heating temperatures of 800 to 1000K were considered favorable when jumping the temperature for only ligands.

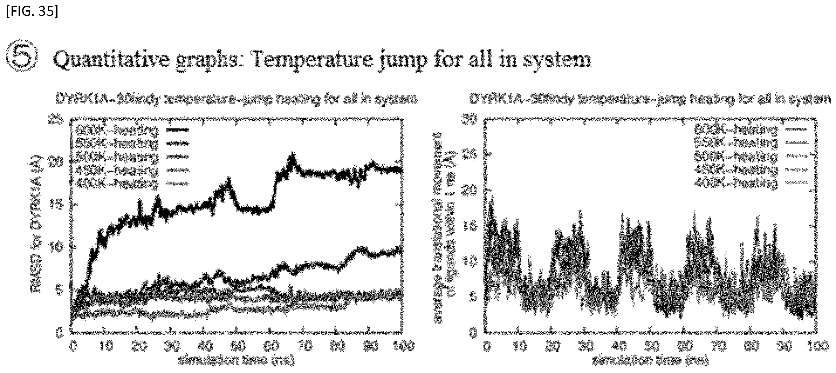

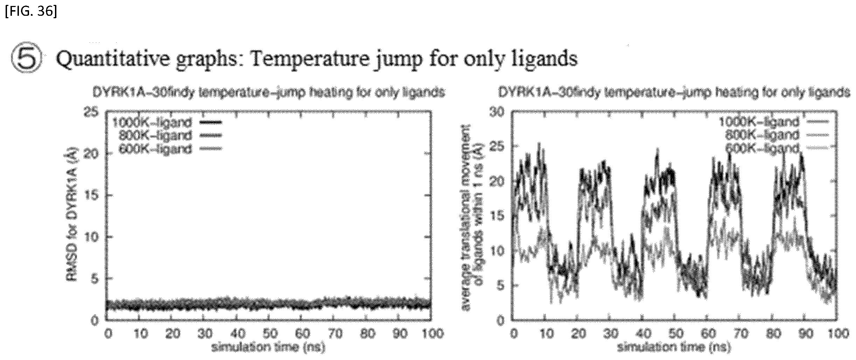

Furthermore, diversity of the structures identified during the calculation period was analyzed. FIG. 35 provides quantitative graphs of jumping the temperature for all in the system, while FIG. 36 provides quantitative graphs of jumping the temperature for only ligands. It should be noted that the RMSD value for DYRK1A is an indicator of deviation in the calculated structure relative to the native state (RMSD: root mean square deviation). Also, the movement of ligands for 1 ns indicates the distance of translational movement made by the ligands for 1 ns.

As shown in FIG. 35 and FIG. 36, the results of calculating the structural fluctuations at the respective temperatures were obtained. Specifically, fluctuation of the structure was confirmed in a temperature-dependent manner. For example, FIG. 35 (temperature jump for all in system) reveals that the DYRK1A structure became very different from the native state when the heating temperature was raised. Meanwhile, FIG. 36 (temperature jump for only ligands) reveals that the ligand dispersibility improved while the DYRK1A structure remained intact.

This completes the explanation of the embodiment including the Examples.

According to the aforementioned embodiment, the huge gap between the compound diversity and the number of protein binding sites can be bridged, to increase the probability of discovering a useful functional substance. Additionally, exposing the potential compound binding sites in proteins that were not targeted under prior art, allows for evaluation of/search for functional effects that are attributable to compounds having structures different from those identified under prior art.

### [Other Embodiments]

The foregoing explained a mode for carrying out the present invention; however, the present invention may be carried out in various different modes, other than the one mentioned above, within the scope of technical concepts described in "What Is Claimed Is."

For example, of the processes explained in the embodiment, all or part of a process explained as being performed automatically may be performed manually, or all or part of a process explained as being performed manually may be performed automatically using known methods.

Also, the present invention, and the embodiment, may be carried out by replacing "search" where stated, with "evaluation/evaluate." Also, the present invention, and the embodiment, may be carried out by replacing the words "inhibition/inhibit," "function(al)," "acceleration/accelerate," "coagulation/coagulate," "stabilization/stabilize," "activation/activate," etc., with one another.

In addition, the processing procedures, control procedures, specific names and information of each process including data, conditions and other parameters that are described or shown in the aforementioned documents or drawings, as well as examples of screens and database configurations that are not shown, may be changed arbitrarily except where specifically noted.

Also, in regards to the selective functional substance search system 100, it is explained, for example, that the system performs processing in a standalone mode; however, this has no limiting effect and it can perform processing in response to a request from a client terminal (such as the user's mobile terminal). Also, each illustrated constituent represents a functional concept and need not be physically constituted as illustrated.

For example, the whole or any part thereof, of any processing function provided by each unit, especially each processing function executed by the control part 102, may be implemented by a CPU (central processing unit) and a program interpreted and executed by the CPU, or as wired-logic hardware. It should be noted that the program, including programmed instructions for instructing a computer to execute the method pertaining to the present invention, is recorded in a non-transitory, computer-readable recording medium, and, if necessary, mechanically read into an electronic control unit. In other words, a computer program that cooperates with the OS (operating system) to give instructions to the CPU and perform various processing is recorded, for example, in the memory part 106 being a ROM, HDD (hard disk drive), etc. This computer program is loaded into a RAM to be executed, and constitutes the control part cooperatively with the CPU.

Also, this computer program may be stored in an application program server connected to the system via an arbitrary network, and can be downloaded, in whole or in part, as necessary.

Additionally, the program pertaining to the present invention may be stored in a computer-readable recording medium, or it may be constituted as a program product. Here, this "recording medium" includes any "portable physical medium" such as memory card, USB memory, SD card, flexible disk, magneto-optical disc, ROM, EPROM, EEPROM, CD-ROM, MO, DVD, Blu-ray (registered trademark) disc, etc.

Meanwhile, the "program" is a data processing method described in an arbitrary language or description method, and its source code, binary code, etc., can be in any format. Also, the "program" is not necessarily limited to one configured in a unitary manner, and includes one configured as multiple modules and libraries in a distributed manner or one that achieves its function by cooperating with a separate program, a representative example of which is an OS (operating system). It should be noted that, in each unit described in an embodiment, any known configuration or procedure may be used, for example, for the specific configuration and reading procedure for reading recording media or the subsequent installation procedure. The present invention may be constituted as a program product recorded in a non-transitory, computer-readable recording medium.

As for the various files, databases, etc., stored in the memory part 106, they are RAM, ROM or other memory unit, hard disk or other fixed disk unit, flexible disk, optical disc or other storage means, in which various programs, tables, databases, webpage files, etc., used in various processing and for providing websites are stored.

Also, the selective functional substance search system 100 may be constituted as any known personal computer, workstation, mobile device, smartphone or other information processing unit, or it may be constituted as such information processing unit to which arbitrary peripherals are connected. Also, the system 100 may be implemented by installing software (including program, data, etc.) for instructing the information processing unit to implement the method pertaining to the present invention.

Furthermore, the specific mode of system distribution/integration is not limited to what is illustrated, and the system may be constituted by functionally or physically distributing/integrating all or part of the system in arbitrary constitutional units according to various additions, etc., or according to the functional loads. In other words, the aforementioned embodiments may be implemented in any desired combination, or any embodiment may be implemented selectively.

### Industrial Field of Application

As explained in detail above, a method of searching for selective functional substances, a selective functional substance search system, a selective functional substance search method, and a program, that can bridge the huge gap between the diversity of compounds and the number of protein binding sites to increase the probability of discovering a useful functional substance, can be provided according to the present invention. To be specific, an art of diversifying the structure of the target protein and thereby expanding the matching patterns with compounds, for the purpose of improving the hit ratio in compound search, can be provided, which will facilitate search for useful compounds in various fields and thus contribute to extending our healthy life expectancy and creating new markets through search for and discovery of these useful compounds.

### Description of the Symbols

- 100: Selective functional substance search system
- 102: Control part
- 102a: Destabilization part
- 102b: Determination part
- 102c: Device control part
- 104: Communication control interface part
- 106: Memory part
- 106a: Structure file
- 106b: Evaluation file
- 108: Input/output control interface
- 112: Input part
- 114: Output part
- 200: External device
- 300: Network

## Claims

1. A method of searching for selective functional substances to search for functional substances that selectively bind to a non-native state of a protein and influence a function of the protein, the method of searching for selective functional substances including:
a step to destabilize the protein at least partially;
a step to induce restabilization by providing the destabilized protein in a presence of a candidate functional substance; and
a step to determine an effect of the presence of the candidate functional substance in influencing the function of the protein.

2. The method of searching for selective functional substances according to claim 1, wherein, in the step to destabilize, a solution containing the protein is subjected to a temperature change, a pressure change, a pH change, an addition of denaturant, or an alteration of electric-charge on the protein.

3. The method of searching for selective functional substances according to claim 1 or 2, wherein the protein is an enzyme such as kinase or protease, photoprotein, other functional protein.

4. The method of searching for selective functional substances according to claim 2, wherein the step to destabilize the protein implements a temperature change, and a destabilizing temperature is 50°C to 70°C.

5. The method of searching for selective functional substances according to claim 4, wherein the protein is destabilized by means of the temperature change for a specified heating temperature period, and/or a cooling process is performed as the step to induce restabilization.

6. The method of searching for selective functional substances according to any one of claims 1 to 5, wherein the function of the protein is exhibited in a presence of a substrate or binding substance.

7. The method of searching for selective functional substances according to any one of claims 1 to 6, wherein an activity of the candidate functional substance with respect to the destabilized protein is detected in a dynamic range of 1.5 times or more in a presence of a surfactant or hydrotrope.

8. The method of searching for selective functional substances according to any one of claims 1 to 7, wherein the candidate functional substance is a small molecule, middle molecule, large molecule, peptide, antibody, or nucleic-acid aptamer.

9. The method of searching for selective functional substances according to any one of claims 1 to 8, wherein the functional substance is an inhibitor, accelerator, coagulant, stabilizer, or activator of the protein.

10. A selective functional substance search system comprising at least a memory part and a control part, to search for functional substances that selectively bind to a non-native state of a protein and influence a function of the protein, the selective functional substance search system **characterized in that**:
the memory part stores structure data relating to at least one candidate functional substance; and
the control part comprises:
a destabilization part that destabilizes the protein at least partially and provides the destabilized protein in a presence of the candidate functional substance, through simulation; and
a determination part that determines an effect of the presence of the candidate functional substance in influencing the function of the protein.

11. A selective functional substance search method to be executed by a computer comprising at least a memory part and a control part, in order to search for functional substances that selectively bind to a non-native state of a protein and influence a function of the protein, the selective functional substance search method **characterized in that**:
the memory part stores structure data relating to at least one candidate functional substance; and
the method includes the following executed in the control part:
a destabilization step to destabilize the protein at least partially and provide the destabilized protein in a presence of the candidate functional substance, through simulation; and
a determination step to determine an effect of the presence of the candidate functional substance in influencing the function of the protein.

12. A program to be executed by a computer comprising at least a memory part and a control part, in order to search for functional substances that selectively bind to a non-native state of a protein and influence a function of the protein, wherein:
the memory part stores structure data relating to at least one candidate functional substance; and
the program is designed to execute in the control part:
a destabilization step to destabilize the protein at least partially and provide the destabilized protein in a presence of the candidate functional substance, through simulation; and
a determination step to determine an effect of the presence of the candidate functional substance in influencing the function of the protein.
